# EUROPEAN PATENT APPLICATION

(11) **EP 4 722 242 A1**
(43) Date of publication of application: **08.04.2026**
(21) Application number: 24815544.2
(22) Date of filing: 30.05.2024
(51) Int. Cl.: C07K 16/18, A61K 39/395, A61K 45/00, A61K 47/68, A61K 49/00, A61P 21/00, A61P 25/02, A61P 43/00, C12N 1/15, C12N 1/19, C12N 1/21, C12N 5/10, C12N 5/0793, C12N 15/13

(54) **ANTI-SYNAPTOTAGMIN 2 ANTIBODY**

(30) Priority: 31.05.2023 JP 2023090263
(71) Applicant: Jiksak Bioengineering, Inc., Kawasaki-shi, Kanagawa 210-0821 (JP)
(72) Inventor: YUMOTO, Norihiro, Kawasaki-shi, Kanagawa 210-0821 (JP)
(74) Representative: Mewburn Ellis LLP
(86) International application number: PCT/JP2024/019803
(87) International publication number: WO 2024/248063

(57) **Abstract**

The problem addressed by the present invention is to provide an antibody that excels at binding to the intravesicular domain of synaptotagmin 2.

Provided is a synaptotagmin 2 antibody capable of efficiently targeting a desired substance toward motor neuron synapses.

## Description

### Technical Field

The present invention relates to a monoclonal antibody that binds to the intravesicular domain of Synaptotagmin 2. The antibody according to the present invention can be used as a targeting agent for delivering a physiologically active substance and/or a label substance to a motor neuron, and a pharmaceutical composition comprising the targeting agent.

### Background Art

The nerve is composed of central nerves and peripheral nerves, and regulates various emotions, functions of various organs such as muscles and internal organs, and the like. Such neural functions may be impaired due to nerve damage, disease, aging, and the like, and in such cases, mental and physical health may be impaired and may have great effects in the course of social life. Therefore, maintaining and improving neural function is an extremely critical issue because it is directly linked to maintaining and improving the quality of life (QOL).

The central nerves and peripheral nerves are each composed of neurons, which exchange signals with each other via synapse. Synapse is a junction that comprises a cleft formed between the axon terminal of a neuron (the presynapse) and dendrites of another neuron or cells such as skeletal muscles or organs (the postsynapse), and transmits signals through binding of chemicals released from the presynapse to receptors located on the postsynapse. Synaptogenesis (synapse formation) is triggered by the interaction of specific membrane proteins expressed on the presynapse and postsynapse.

In recent years, compounds and peptides capable of maintaining and improving neural function by promoting synaptogenesis have been developed. Patent Literature 1 discloses that certain peptides have a dendritic elongation-promoting function and a synaptogenesis-promoting function in primary cultured cortical neuronal cells (PCN), and that such peptides are used for the treatment of mild cognitive impairment or early dementia. Patent Literature 2 discloses that C-terminal fragment β (CTFβ) generated by cleavage of amyloid precursor protein (APP) by β-secretase promotes synaptogenesis, and that CTFβ is used for the treatment of neurodegenerative diseases or the like.

In addition, Patent Literature 3 discloses methods for culturing motor neurons with presynapse using microbeads with an LRRTM molecule or a fusion protein comprising the molecule having immobilized thereon. In this way, it is expected that a number of compounds and drug candidates capable of acting on motor neurons will be provided in the future by developing a technique that can easily screen the influences of drugs on the function of motor neurons in vitro.

### Citation List

### Patent Literature

Patent Literature 1: JP 2012-092048 A
Patent Literature 2: JP 2008-143867 A
Patent Literature 3: WO2021/006075

### Summary of Invention

### Technical Problem

The present inventors have developed a method that achieves controlled transport of drugs to a target motor neuron by utilizing an antibody capable of binding to a membrane protein present in the synaptic vesicle of the motor neuron (WO 2023/210585). With this method, the compounds or drugs as described above can be allowed to act on the motor neuron. However, there has existed no excellent antibody capable of binding to such a membrane protein because such a method of utilization has not been expected therebefore.

Thus, the object of the present invention is to provide an excellent monoclonal antibody capable of binding to a membrane protein present in the synaptic vesicle of a motor neuron, and to provide a means for targeting a substance to a motor neuron and a means for visualizing a targeting site using the same.

### Solution to Problem

As a result of intensively studying to solve the above problem, the present inventors successfully obtained an excellent monoclonal antibody that binds to the intravesicular domain (N-terminal portion) of Synaptotagmin 2 and found that a desired substance can be efficiently targeted to a motor neuron synapse by using this antibody, and thus the present invention has been completed.

The present invention has been made on the basis of these findings and provides the following:
[1] an antibody capable of binding to the intravesicular domain of Synaptotagmin 2, the antibody comprising a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.
[2] the antibody according to [1], wherein the antibody is selected from the following (a) to (c): (a) an antibody having the heavy chain variable region consisting of the amino acid sequence having the sequence identity of 90% or more to SEQ ID NO: 1, and the light chain variable region consisting of the amino acid sequence having the sequence identity of 90% or more to SEQ ID NO: 2; (b) an antibody having the heavy chain variable region consisting of the amino acid sequence having substitution, deletion, and/or addition of one or several amino acids in SEQ ID NO: 1, and the light chain variable region consisting of the amino acid sequence having substitution, deletion, and/or addition of one or several amino acids in SEQ ID NO: 2; and (c) an antibody having the heavy chain variable region consisting of the amino acid sequence of SEQ ID NO: 1, and the light chain variable region consisting of the amino acid sequence of SEQ ID NO: 2.
[3] a conjugate of the antibody according to [1] or [2] and a label substance and/or a physiologically active substance.
[4] a nucleic acid molecule having a nucleotide sequence encoding the antibody according to [1] or [2].
[5] a cell comprising the nucleic acid molecule according to [4].
[6] a targeting agent to a motor neuron comprising the antibody according to [1] or [2].
[7] the targeting agent according to [6], further comprising a label substance and/or a physiologically active substance.
[8] the targeting agent according to [7], comprising a conjugate of the antibody and the label substance and/or the physiologically active substance.
[9] the targeting agent according to [7] or [8], wherein the label substance is a fluorescent molecule.
[10] the targeting agent according to any of [7] to [9] comprising the label substance, wherein the targeting agent is a motor neuron visualizing agent.
[11] the targeting agent according to any of [7] to [10], wherein the physiologically active substance is one or more selected from the group consisting of a synaptogenesis-promoting agent, a synapse-maintaining agent, a muscle-enhancing agent, and a neuronal function-modifying agent.
[12] the targeting agent according to any of [6] to [11], wherein the targeting agent is incorporated intracellularly by endocytosis.
[13] a pharmaceutical composition comprising the targeting agent according to any of [7] to [12].
[14] a method for targeting the label substance and/or the physiologically active substance to a motor neuron comprising a step of contacting the targeting agent according to any of [7] to [12] and/or the pharmaceutical composition according to [13] with a motor neuron; and a step of delivering the targeting agent and/or the pharmaceutical composition to a synapse of the motor neuron.
[15] a method for visualizing a motor neuron comprising a step of contacting the targeting agent which is the motor neuron visualizing agent according to [10] with a motor neuron; a step of delivering the targeting agent to a synapse of the motor neuron; and a step of detecting a signal of the label substance.
[16] a method for preventing or treating a condition or a disease comprising a step of contacting the targeting agent according to any of [7] to [12] with a motor neuron; and a step of delivering the targeting agent to a synapse of the motor neuron.
[17] the method for preventing or treating according to [16], wherein the condition or disease is a condition or disease exhibiting impaired neural function.
[18] the method for preventing or treating according to [16] or [17], wherein the condition or disease is a neurological disease and a neuromuscular disease.
[19] the method for preventing or treating according to any of [16] to [18], wherein the targeting agent comprises a conjugate of the antibody and a physiologically active substance.
[20] use of an antibody capable of binding to the intravesicular domain of Synaptotagmin 2 in the preparation of a medicament comprising the antibody and a physiologically active substance, the antibody comprising a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.
[21] a composition for targeting to a motor neuron comprising an antibody capable of binding to the intravesicular domain of Synaptotagmin 2, the antibody comprising a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.
[22] an antibody capable of binding to the intravesicular domain of Synaptotagmin 2 for use in a method for preventing or treating a condition or a disease, the antibody comprising a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.
[23] an antibody capable of binding to the intravesicular domain of Synaptotagmin 2 for use in method for targeting a label substance and/or a physiologically active substance to a motor neuron, the antibody comprising a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.

The present specification encompasses the contents disclosed in Japanese Patent Application Nos. 2023-090263, on which the priority of the present application is based.

### Advantageous Effects of Invention

The present invention provides a monoclonal antibody that can target a desired substance to a motor neuron. When the substance is a physiologically active substance or a therapeutic agent, conditions and/or diseases associated with motor neuron disorders can be treated. Additionally, where the substance is a label substance, a motor neuron can be visualized.

### Brief Description of Drawings

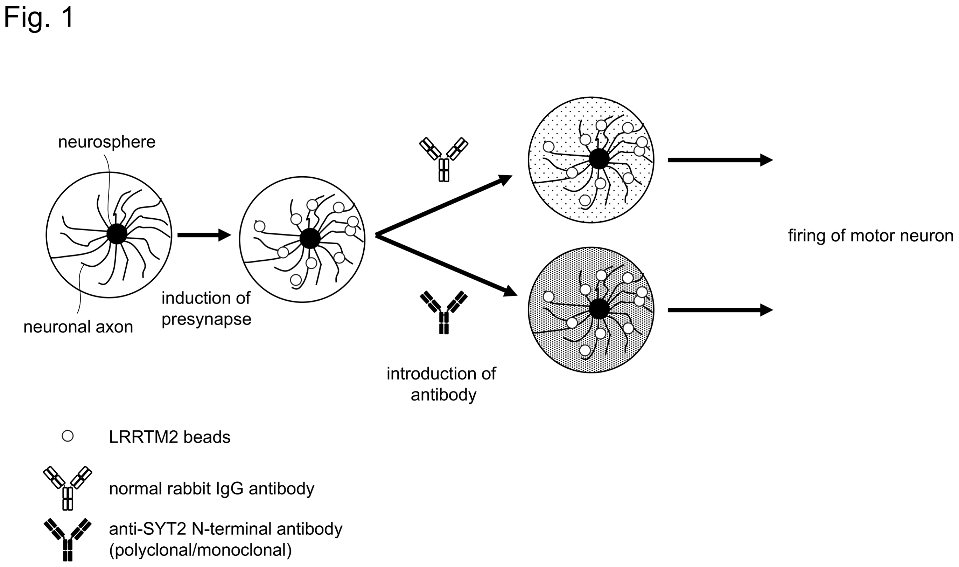

[Figure 1] Figure 1 is a schematic diagram illustrating the experimental procedure for presynapse induction using LRRTM2 beads and antibody delivery. When a neurosphere is seeded on the plate, the neuronal axons are elongated from the neurosphere. By subsequently seeding LRRTM2 beads therein, a presynapse is induced from the elongated neuronal axon to the surface of the LRRTM2 bead. On the plate in which presynapse was induced in this way, a normal rabbit IgG antibody (control) or an anti-Synaptotagmin 2 intravesicular domain (N-terminal) antibody (anti-SYT2 N-terminal antibody (polyclonal antibody or the monoclonal antibody according to the present invention)) is introduced to examine whether the anti-SYT2 N-terminal antibody can be targeted to the presynapse by firing neurons.

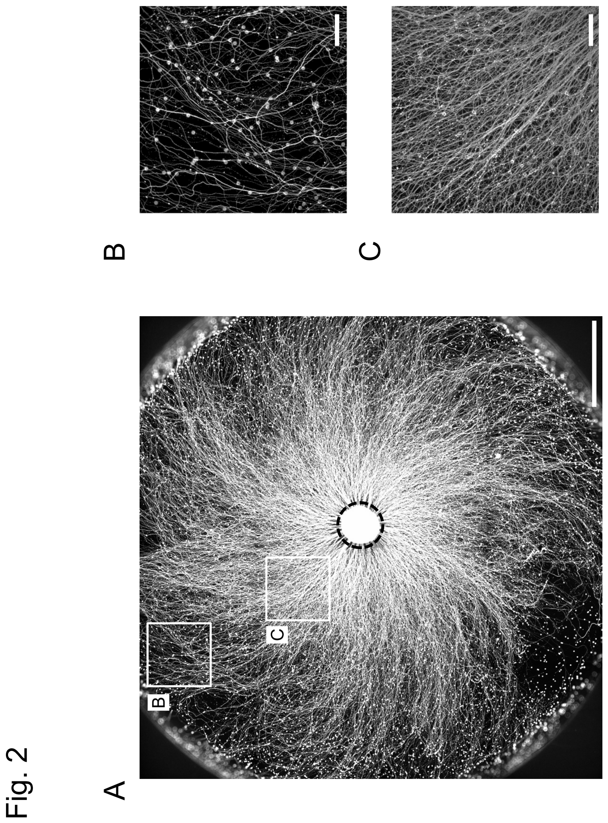

[Figure 2] Figure 2 shows fluorescent images of the control antibody group in which the conjugate of normal rabbit antibody and monomethyl auristatin E (MMAE) was introduced. The fluorescent signal shown in the figure is a signal based on βIII-tubulin (Tuj1). In Figure A, the black dashed circle indicates the position of the neurosphere. Figures B and C show magnified images of the white frame area in Figure A. In Figure A, the scale bar represents 1mm, and in Figures B and C, scale bars represent 100µm.

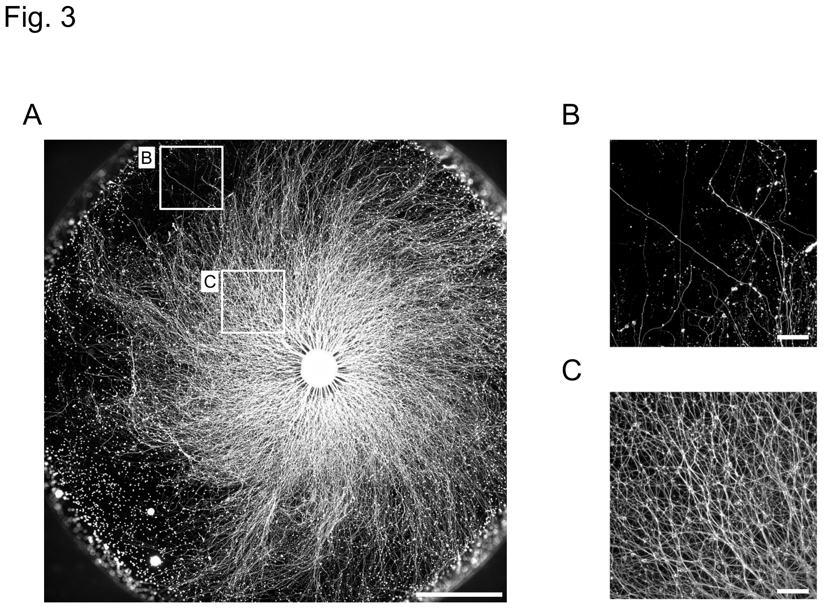

[Figure 3] Figure 3 shows fluorescent images of the SYT2 antibody group in which the conjugate of anti-Synaptotagmin 2 intravesicular domain (N-terminal) polyclonal antibody (polyclonal anti-SYT2 N-terminal antibody) and MMAE was introduced. The fluorescent signal shown in the figure is a signal based on Tuj1. Figures B and C show magnified images of the white frame area in Figure A. In Figure A, the scale bar represents 1mm, and in Figures B and C, scale bars represent 100µm.

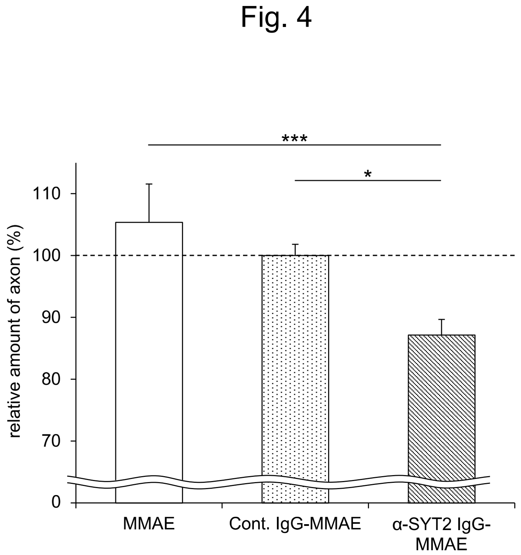

[Figure 4] Figure 4 shows a graph in which the pharmacological effects based on MMAE were quantified. In the figure, the relative amount of axon is the value normalized to the results obtained using the conjugate of the control normal rabbit antibody and MMAE ("Cont. IgG-MMAE" in the figure), set to 100%. In the figure, the dashed line indicates the position where the relative amount of axon is 100%, "MMAE" indicates the result of the simple introduction group in which MMAE was introduced alone, and "α-SYT2 IgG-MMAE" indicates the result of the SYT2 antibody group in which the conjugate of polyclonal anti-SYT2 N-terminal antibody and MMAE was introduced. In the figure, error bars indicate standard error, "*" indicates p<0.05, and "***" indicates p<0.001.

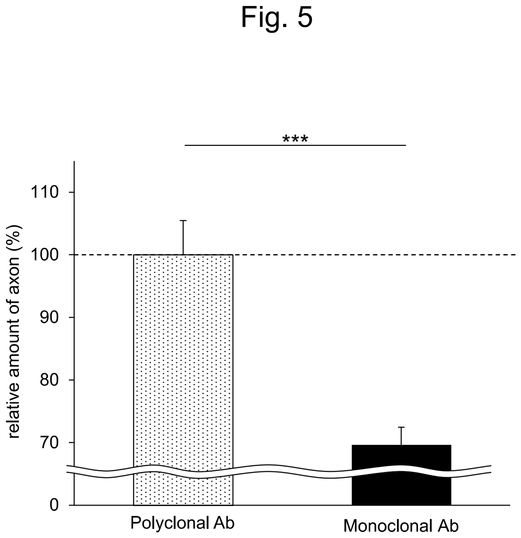

[Figure 5] Figure 5 shows a graph in which the pharmacological effects based on MMAE were quantified. In the figure, the relative amount of axon is the value normalized to the results obtained using the conjugate of the polyclonal anti-SYT2 N-terminal antibody and MMAE ("Polyclonal Ab" in the figure), set to 100%. In the figure, the dashed line indicates the position where the relative amount of axon is 100%, and "Monoclonal Ab" indicates the result of the SYT2 antibody group in which the conjugate of anti-Synaptotagmin 2 intravesicular domain (N-terminal) monoclonal antibody (the antibody according to the present invention) and MMAE was introduced. In the figure, error bars indicate standard error, and "**" indicates p<0.01.

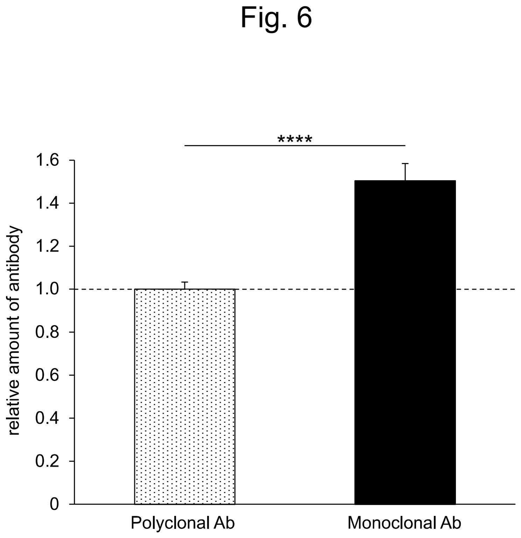

[Figure 6] Figure 6 shows a graph in which the binding affinity of the anti-SYT2 antibody according to the present invention used at 100 ng/mL for Synaptotagmin 2 was quantified by ELISA. In the figure, the relative amount of antibody is the value normalized to the results obtained using the polyclonal anti-SYT2 N-terminal antibody ("Polyclonal Ab" in the figure), set to 1.0. In the figure, the dashed line indicates the position where the relative amount of antibody is 1.0, and "Monoclonal Ab" indicates the results obtained using the antibody according to the present invention. In the figure, error bars indicate standard error, and "****" indicates p<0.0001.

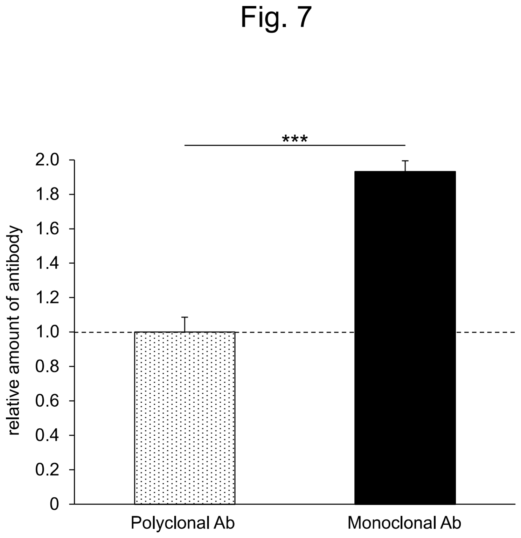

[Figure 7] Figure 7 shows a graph in which the binding affinity of the anti-SYT2 antibody according to the present invention used at 500 ng/mL for Synaptotagmin 2 was quantified by ELISA. In the figure, the relative amount of antibody is the value normalized to the results obtained using the polyclonal anti-SYT2 N-terminal antibody ("Polyclonal Ab" in the figure), set to 1.0. In the figure, the dashed line indicates the position where the relative amount of antibody is 1.0, and "Monoclonal Ab" indicates the results obtained using the antibody according to the present invention. In the figure, error bars indicate standard error, and "****" indicates p<0.0001.

### Description of Embodiments

The present invention provides a monoclonal antibody that is capable of binding to the intravesicular domain (N-terminal portion) of Synaptotagmin 2 and can be used for targeting a substance of interest to a motor neuron (referred to as the "antibody according to the present invention").

### < Synaptotagmin 2 >

As used herein, "Synaptotagmin 2" refers to one of the membrane proteins belonging to the Synaptotagmin family. Synaptotagmin family encompasses 17 proteins in mammals, of which Synaptotagmin 2 is a protein that is expressed primarily on the synaptic vesicle membrane of the presynapse of the neuromuscular junction in peripheral nerves and promotes the fusion of synaptic vesicle with the plasma membrane in a calcium-ion dependent manner (see, for example, Rickman, Colin, et al., Journal of Biological Chemistry 279.13 (2004): 12574-12579., Stephanie Bauche, et al., Neurol Genet. 2020 Dec 3; 6(6): e534. doi: 10.1212). Synaptotagmin 2 is a single-spanning transmembrane protein and comprises, in order from the N-terminus, an intravesicular domain, a transmembrane domain, and a cytoplasmic domain. It is known that the C-terminal cytoplasmic domain has a tandem C2 domain capable of binding calcium ions and that this cytoplasmic domain is mainly responsible for the function related to the fusion of membranes.

The intravesicular domain of Synaptotagmin 2 is exposed to the lumen of the synaptic vesicle. When the synaptic vesicle and the plasma membrane fuse with each other as the intracellular calcium-ion level increases, the lumen of the synaptic vesicle is connected to the extracellular space, and the intravesicular domain of Synaptotagmin 2 is temporarily exposed to the extracellular space. Thereafter, the cellular membrane portion containing Synaptotagmin 2 is recovered as a synaptic vesicle membrane into the cells by endocytosis and reused as a synaptic vesicle. At this time, the intravesicular domain of Synaptotagmin 2 is exposed to the lumen of the synaptic vesicle again.

Specifically, an exemplary human Synaptotagmin 2 is a protein consisting of 419 amino acids with an amino acid sequence of SEQ ID NO: 9. For example, in SEQ ID NO: 9, the position of each domain is as follows: the intravesicular domain is a region having an amino acid sequence from position 1 to position 62; the transmembrane domain is a region having an amino acid sequence from position 63 to position 83; and the cytoplasmic domain is a region having an amino acid sequence from position 84 to position 419.

An exemplary mouse Synaptotagmin 2 is a protein consisting of 422 amino acids with an amino acid sequence of SEQ ID NO: 10. In SEQ ID NO: 10, the position of each domain is as follows: intravesicular domain is a region having an amino acid sequence from position 1 to position 60; the transmembrane domain is a region having an amino acid sequence from position 61 to position 87; and the cytoplasmic domain is a region having an amino acid sequence from position 88 to position 422.

The sequence information of Synaptotagmin 2 of other organisms can readily be obtained from known databases, such as NCBI databases.

As used herein, "intravesicular domain of Synaptotagmin 2" refers to the entire or a segment of the intravesicular domain present in the N-terminal portion of Synaptotagmin 2. The entire intravesicular domain includes, for example, a region having an amino acid sequence from position 1 to position 62 in SEQ ID NO: 9 (SEQ ID NO: 11) and/or an amino acid sequence from position 1 to position 60 in SEQ ID NO: 10 (SEQ ID NO: 12). Additionally, a segment of the intravesicular domain includes, for example, a region having a partial sequence with any length from SEQ ID NO: 11 and/or 12, in particular, the region having the amino acid sequence from position 1 to position 25 in SEQ ID NO:9 (SEQ ID NO: 13).

As an antigenic peptide for use in the obtainment of the antibody, a peptide additionally comprising other amino acids in the amino acid sequence of the intravesicular domain or a segment thereof may be used as long as the antibody capable of binding to the intravesicular domain can be obtained in the end. Examples of such an antigenic peptide that may be used include peptides having the amino acid sequences of SEQ ID NOs: 14 to 16.

### < Antibody According to the Present Invention >

The antibody according to the present invention is a monoclonal antibody capable of specifically recognizing and binding to the intravesicular domain (N-terminal portion) of Synaptotagmin 2 (referred to as the "antibody according to the present invention"). The antibody according to the present invention binds to the intravesicular domain of Synaptotagmin 2, but preferably does not bind to the intravesicular domain of other membrane proteins belonging to the Synaptotagmin family (e.g., Synaptotagmin 1). Additionally, the antibody according to the present invention binds to the intravesicular domain of human Synaptotagmin 2 and/or mouse Synaptotagmin 2, and preferably should not bind to the intravesicular domain of Synaptotagmin 2 derived from other animals. Alternatively, the antibody of the present invention binds to the amino acid sequence of SEQ ID NO: 13 in the intravesicular domain of human Synaptotagmin 2, and should not bind to amino acid sequences in other parts of the intravesicular domain of human Synaptotagmin 2.

As used herein, "bind" and "specifically bind" may mean that, but not particularly limited to, the binding between an antigen and an antibody has binding affinity with a KD value of 10⁻⁸ M or less, preferably 10⁻⁹ M or less, more preferably 10⁻¹⁰ M or less (6 × 10⁻¹¹ M or less, 5 × 10⁻¹¹ M or less, 4.5 × 10⁻¹¹ M or less, or the like). The antibody according to the present invention has the binding affinity described above for the intravesicular domain of Synaptotagmin 2. It is preferred that the antibody according to the present invention does not have the binding affinity described above for other antigens comprising other proteins (e.g., the intravesicular domain of other membrane proteins belonging to the Synaptotagmin family, such as Synaptotagmin 1). Alternatively, the binding affinity may be determined by a large association rate constant. In this case, a specific value of the association rate constant is not particularly limited, but may be, for example, 10³/Ms or more, 10⁴/Ms or more, or 10⁵/Ms or more (2 × 10⁵/Ms or more, 2.5 × 10⁵/Ms or more, or the like). Alternatively, the binding affinity may be determined by a small dissociation rate constant. In this case, a specific value of the dissociation rate constant is not particularly limited, but may be, for example, 10⁻³/s or less, 10⁻⁴/s or less (5 × 10⁻⁵/s or less, 2 × 10⁻⁵/s or less, 1.5 × 10⁻⁵/s or less, or the like). Examples of methods for measuring the binding affinity, the association rate constant, and the dissociation rate constant include the surface plasmon resonance method as well as biolayer interferometry. For example, biolayer interferometry may be suitably used.

As used herein, "specifically bind to a target substance" may mean that binding to the target substance, for example, the intravesicular domain of Synaptotagmin 2, is 2 or more times, 3 or more times, or 4 or more times the binding to a substance other than the target substance, for example, when detected by general binding assay using an excessive amount of the target substance. The phrase may mean that an S/N ratio is 2 or more, 3 or more, or 4 or more when the binding is detected from the fluorescent label as described above.

The antibody according to the present invention may be any of a non-human (mouse, rabbit, goat, or the like) antibody, a chimeric antibody, a humanized antibody, and a human antibody, but is preferably, but not particularly limited to, a humanized antibody or a human antibody when used in the treatment of a condition or a disease in a human. Thus, the antibody that may be suitably used in the present invention may be an antibody comprising framework regions (FRs) of a human antibody, and complementarity determining regions (CDRs) capable of bringing about high binding affinity for the intravesicular domain of Synaptotagmin 2.

The antibody according to the present invention may be an IgG antibody molecule, or an antigen-binding fragment or an antigen-binding derivative thereof. For example, an antibody may be a complete antibody, Fab, Fab', F(ab')₂ fragment, or a single-chain antibody (scFv) fragment, in which the heavy chain variable region (VH) and the light chain variable region (VL) are linked via a linker, scFv-Fc, sc(Fv)₂, Fv, diabody, or the like.

scFv, scFv-Fc, and sc(Fv)₂ are synthetic polypeptides in which the variable regions are linked via a linker. The linker may be any one commonly used in the art, and is not particularly limited, but, for example, a peptide linker composed of 5 to 25, preferably 10 to 20 amino acid residues, such as a GS linker, may be suitably used.

The antibody according to the present invention further includes a derivative that can be understood by a person skilled in the art, as long as it should not affect antigen-binding properties, such as a derivative with a modification for facilitating an antibody purification or enhancing the stability, and complexes bound to drugs such as anticancer agents. As used herein, for the sake of convenience, "antibody" is intended to include a fragment and a derivative that retains the binding capacity to the intravesicular domain of Synaptotagmin 2, unless otherwise specified by the context.

The antibody according to the present invention may also be synthesized as a multimer, such as a dimer, a trimer, or a tetramer. Furthermore, the antibody according to the present invention may be a bispecific antibody having first specificity that attains binding to the intravesicular domain of Synaptotagmin 2 and second specificity that attains binding to another antigen. The second specificity may be, for example, for another antigen that can be expressed by a target cell, or may be for the physiologically active substance and/or label substance to be delivered, and can be selected as appropriate depending on the purpose. When the second specificity is for another antigen that can be expressed by a target cell, that is, a motor neuron, the use of the bispecific antibody brings about delivery to the target cell more specifically. When the second specificity is for the physiologically active substance and/or label substance, the substance can be administered together with the bispecific antibody and thereby delivered as a complex to a target site.

A person skilled in the art can obtain the antibody according to the present invention in a form appropriate for use on the basis of the description of the present specification and technical common sense in the art.

Examples of the antibody that binds to the intravesicular domain of Synaptotagmin 2 according to the present invention may include, but are not particularly limited to, the following antibody:
an antibody comprising
a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and
a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.

In this case, the amino acid sequences of other regions (e.g., the amino acid sequences of FRs and the amino acid sequences of constant regions) according to the present invention are not particularly limited. The binding characteristics of an antibody are basically determined depending on the heavy chain and light chain CDRs. Thus, although an amino acid sequence in the antibody according to the present invention may be modified, it is preferred that the modification reside in a framework region or a constant region, from the viewpoint of rarely influencing the binding characteristics. It is also preferred that the modification should not largely change the conformation of the antibody.

Examples of the antibody that binds to the intravesicular domain of Synaptotagmin 2 according to the present invention also include the following antibody:
(a) an antibody having the above HCDR1 to HCDR3 and LCDR1 to LCDR3, wherein the heavy chain variable region consists of the amino acid sequence having the sequence identity of 90% or more to SEQ ID NO: 1, and the light chain variable region consists of the amino acid sequence having the sequence identity of 90% or more to SEQ ID NO: 2.

As used herein, "sequence identity" (of an amino acid sequence) refers to the ratio (%) of the number of matched amino acid residues to the total number of amino acid residues when the amino acid sequences of two polypeptides to be compared are aligned by optionally inserting a gap as appropriate to one or both of the amino acid sequences so as to maximize the number of matches in amino acid residues. The sequence identity can be calculated using an approach and software well known in the art.

In this context, the sequence identity of an amino acid sequence in the antibody according to the present invention is not particularly limited as long as it is 90% or more. The amino acid sequence of the heavy chain variable region consists of an amino acid sequence having the sequence identity of, for example, 90% or more, 91% or more, 92% or more, 93% or more, 94% or more, 95% or more, 96% or more, 97% or more, 98% or more, or 99% or more to SEQ ID NO: 1. Also, the amino acid sequence of the light chain variable region of the antibody according to the present invention consists of an amino acid sequence having the sequence identity of, for example, 90% or more, 91% or more, 92% or more, 93% or more, 94% or more, 95% or more, 96% or more, 97% or more, 98% or more, or 99% or more to SEQ ID NO: 2.

Examples of the antibody that binds to the intravesicular domain of Synaptotagmin 2 according to the present invention also include the following antibody:
(b) an antibody having the above HCDR1 to HCDR3 and LCDR1 to LCDR3, wherein the heavy chain variable region consists of the amino acid sequence having substitution, deletion, and/or addition of one or several amino acids in SEQ ID NO: 1, and the light chain variable region consists of the amino acid sequence having substitution, deletion, and/or addition of one or several amino acids in SEQ ID NO: 2.

In this context, "several" may be 5 or less, for example, 2 or more, 3 or more, 4 or more, or 5. Thus, the antibody according to the present invention may have modification of 5 or less (e.g., 2 or 3) amino acids in each of the heavy chain variable region and/or light chain variable region of the antibody (b). An antibody functionally equivalent to a certain antibody can be prepared by an approach well known in the art, for example, site-directed mutagenesis.

As used herein, "substitution of an amino acid" refers to substitution between 20 types of amino acids constituting a natural protein. The substitution of an amino acid may be, but is not particularly limited to, conservative substitution. The conservative substitution refers to substitution within a conservative amino acid group similar in properties such as charge, side chain, polarity, or aromaticity. Examples thereof include substitution within an uncharged polar amino acid group having a low polar side chain (Gly, Asn, Gln, Ser, Thr, Cys, and Tyr), a branched amino acid group (Leu, Val, and Ile), a neutral amino acid group (Gly, Ile, Val, Leu, Ala, Met, and Pro), a neutral amino acid group having a hydrophilic side chain (Asn, Gln, Thr, Ser, Tyr, and Cys), an acidic amino acid group (Asp and Glu), a basic amino acid group (Arg, Lys, His), or an aromatic amino acid group (Phe, Tyr, and Trp).

Examples of the antibody that binds to the intravesicular domain of Synaptotagmin 2 according to the present invention also include the following antibody:
(c) an antibody in which the heavy chain variable region consists of the amino acid sequence of SEQ ID NO: 1, and the light chain variable region consists of the amino acid sequence of SEQ ID NO: 2.

After an antibody against a certain antigen appears in vivo, an antibody with improved affinity for the antigen more than the original antibody is known to appear by a process called "affinity maturation". Thus, on the basis of the amino acid sequence of each antibody specifically disclosed above, an antibody having activity equivalent to the disclosed antibody or improved more than the disclosed antibody, specifically, an antibody improved in binding affinity for the intravesicular domain of Synaptotagmin 2 and functionality as a targeting agent for the delivery of a substance, can be obtained. Such modification may occur within framework regions and constant regions as well as within CDR regions.

A method for obtaining the antibody according to the present invention is not particularly limited. A monoclonal antibody may be obtained by a known approach, for example, by immunizing a non-human mammal with the intravesicular domain of Synaptotagmin 2 identified as an antigen. The antibody according to the present invention may also be obtained by synthesis using a genetic engineering approach or using a chemical synthesis means on the basis of information on the amino acid sequence of the antibody according to the present invention demonstrated to have activity, or information on the nucleotide sequence of a polynucleotide encoding the antibody.

In the case of producing the antibody by the genetic engineering approach, the antibody can be expressed and obtained as a recombinant protein by introducing polynucleotides encoding the heavy chain and the light chain into appropriate host cells. In this case, the polynucleotides may be DNA or may be RNA, and an approach for introduction to host cells used in the art may be utilized as appropriate. As vectors for introducing the polynucleotides into host cells, virus vectors, plasmid vectors, phage vectors, or the like can be used as appropriate. As the host cells, for example, bacteria such as E. coli, yeasts, insect cells, or animal cells may be used. In this context, the polynucleotides encoding the heavy chain and the light chain may be introduced into separate vectors or may be ligated and introduced into the same vector.

For example, the antibody according to the present invention can exhibit excellent physiological activity against motor neurons in the form of a conjugate with a physiologically active substance. Improved physiological activity based on use of the antibody according to the present invention may be confirmed, for example, by coculturing motor neurons with microbeads with an LRRTM molecule immobilized on the surface beforehand, administering monomethyl auristatin E (MMAE) and the conjugate according to the present invention to the resulting motor neurons with axonal elongation and presynapse formation are induced, and using the obtained results as an index. In this respect, for example, the targeting efficiency of the antibody according to the present invention may be confirmed by comparing an axonal amount obtained using a conjugate of a polyclonal anti-SYT2 N-terminal antibody (e.g., an antibody of Cat. No. 105 223, Synaptic Systems) and MMAE with an axonal amount obtained using a conjugate of the antibody according to the present invention and MMAE. Use of the antibody according to the present invention decreases, for example, the axonal amount compared to use of the polyclonal antibody. For example, the axonal amount may be statistically significantly decreased, but may be, specifically, 90% or less, 80% or less, 75% or less, 75% or less, on average, of the axonal amount obtained using the polyclonal antibody.

### < Nucleic Acid Molecule >

The present invention also provides a nucleic acid molecule having a nucleotide sequence encoding the antibody according to the present invention. The nucleic acid molecule according to the present invention may be a nucleic acid molecule encoding any one or more antibodies according to the present invention. The nucleotide sequence of such a nucleic acid molecule is not particularly limited. Examples thereof include codon-optimized nucleotide sequences and nucleotide sequences with a start codon (ATG) added on the 5'-terminal side. The nucleic acid molecule according to the present invention may be DNA, RNA, or a combination thereof and may comprise a natural nucleotide as well as a modified nucleotide, a non-natural nucleotide, and the like as appropriate.

The nucleic acid molecule according to the present invention may comprise any other component in addition to the nucleic acid encoding the antibody according to the present invention. The nucleic acid molecule may further comprise, for example, a promoter to give a gene expression vector that enables the antibody according to the present invention to be expressed in cells.

As used herein, "gene expression vector" refers to a vector that comprises a gene or a gene fragment (hereinafter, referred to as "gene or the like") in an expressible state and encompasses an expression unit that can control the expression of the gene or the like. The gene expression vector may be a plasmid vector, a virus vector, or a phage vector. For example, a plasmid vector that facilitates a gene recombination operation, or a virus vector that can easily introduce the gene into immunocytes may be used. The vector according to the present invention may optionally comprise additional components such as a label gene (selective marker), an enhancer, a terminator, a replication origin, and a polyA signal.

As used herein, "expressible state" means that the gene or the like to be expressed is placed in a region downstream of a promoter under the control of the promoter.

The type of a specific vector is not particularly limited. For example, the plasmid vector may be, for example, a commercially available expression vector for a mammalian cell, such as pCI vector, pSI vector, or pcDNA3 vector from Promega, or a shuttle vector capable of replicating between mammalian cells and bacteria, such as E. coli.

As the virus vector, for example, a virus vector such as a retrovirus vector (including an oncoretrovirus vector, a lentivirus vector, and a pseudo-vector), an adenovirus vector, an adeno-associated virus (AAV) vector, a simian virus vector, a vaccinia virus vector, a Sendai virus vector, an Epstein-barr (EBV) vector, and an HSV vector may be used. A virus vector lacking the ability to replicate so as not to self-replicate in infected cells may be used.

As used herein, "promoter" is a gene expression regulation region that can control the expression of the gene or the like placed downstream (on the 3'-terminal side) in cells harboring the gene expression vector. Promoters can be classified into a ubiquitous promoter (systemic promoter) and a site-directed promoter on the basis of the location where the gene or the like under expression control is expressed. The ubiquitous promoter is a promoter that controls the expression of the gene or the like to be expressed (gene or the like of interest) in all cells, that is, the whole host individual. The site-directed promoter is a promoter that controls the expression of the gene or the like of interest only in certain cells or tissues. The promoter comprised in the gene expression vector according to the present invention may be the ubiquitous promoter or the site-directed promoter, but preferably can induce the expression in host cells.

Promoters are also classified into a promoter with constitutive activity, an expression-inducible promoter, or a promoter with time-specific activity on the basis of the timing of expression. The promoter with constitutive activity enables the gene or the like of interest to be constitutively expressed in cells. The expression-inducible promoter can induce the expression of the gene or the like of interest at any time point in cells. The promoter with time-specific activity can induce the expression of the gene or the like of interest at a certain time point in the stage of development in cells. All the promoters above can allow the overexpression of the gene of interest in host cells and may therefore be interpreted as overexpression-type promoters. The promoter comprised in the gene expression vector according to the present invention is preferably a promoter with constitutive activity that permits long-term sustention of therapeutic effects.

The promoter in the gene expression vector according to the present aspect is a promoter that can induce the expression of the nucleic acid encoding the antibody according to the present invention in host cells. Specific examples thereof include CMV promoter (CMV-IE promoter), SV40 early promoter, RSV promoter, EF1α promoter, Ub promoter, and 5' LTR promoter. In the case of a retrovirus vector, the nucleic acid encoding the antibody according to the present invention may be placed downstream of 5' LTR promoter to induce its gene expression.

### < Cell >

The present invention further provides a cell capable of expressing the antibody according to the present invention.

The type of the host cell according to the present invention is not particularly limited. For example, although an organism from which the cell is derived is not particularly limited, for example, a cell derived from a vertebrate described later for a targeting agent can be used as the cell according to the present invention. The cellular species of the host cell is not particularly limited. For example, ectodermal cells, mesodermal cells, endodermal cells, and a combination thereof may be used. For example, cells of an epithelial tissue, a connective tissue, a cartilage tissue, a bone tissue, a blood tissue (including a lymph tissue), a muscle tissue, a nerve tissue, or a combination thereof may be used. The cell according to the present invention may be a cell having no ability to differentiate or may be a cell having the ability to differentiate (e.g., multipotent cells and pluripotent cells). Specific examples of the cell having the ability to differentiate include mesenchymal stem cells, hematopoietic stem cells, various cancer cell lines, neural stem cells, iPS cells, and ES cells.

As used herein, the term "pluripotent stem cell" refers to a cell that is capable of self-renewal, can be cultured in vitro, and has pluripotency capable of differentiating into cells constituting an individual. Specific examples thereof include an embryonic stem cell (ES cell), a pluripotent stem cell derived from a fetal primordial germ cell (GS cell), an induced pluripotent stem cell derived from a somatic cell (iPS cell), and the like, but a human-derived iPS cell or ES cell is preferably used in the present method.

The ES cell is often obtained from a fertilized egg but can also be obtained from sources other than a fertilized egg, such as adipose tissue, placenta, a testicular cell, and any ES cell is the subject of the present invention. The methods for producing an ES cell from other than a fertilized egg have been reported (e.g., WO2003/046141), and these reports can be referred to and applied as appropriate.

The iPS cell is an artificial stem cell derived from somatic cells, which can be produced by introducing specific reprogramming factors into a somatic cell in the form of nucleic acids or proteins, and exhibits properties almost comparable to those of the ES cell (e.g., pluripotency and proliferative capacity based on self-renewal). Examples of genes encompassed in the reprogramming factors include Oct3/4, Sox2, Sox1, Sox3, Sox15, Sox17, Klf4, Klf2, c-MYC, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, or a combination thereof. Many reports for methods for producing an iPS cell are provided, and these reports may be referred to, and may be appropriately modified and applied. The iPS cell that can be used in the present method is preferably a human-derived iPS cell, for example, a human fibroblast-derived iPS cell.

Many reports for methods for inducing differentiation of human iPS cells into peripheral neurons have been provided (e.g., Chambers, Stuart M., et al., Nature biotechnology 27.3 (2009): 275.), and these reports may be referred to, and may be appropriately modified and applied. Alternatively, various neural cells into which a human iPS cell is induced to differentiate are commercially available and can be purchased, for example, from FUJIFILM Cellular Dynamics, Inc., Reprocell Inc., or the like.

The cell according to the present invention is capable of expressing the antibody according to the present invention. Preferably, the cell according to the present invention comprises the nucleic acid molecule according to the present invention. The cell according to the present invention can comprise multiple copies of the nucleic acid molecule according to the present invention.

### < Targeting Agent >

The present invention also relates to a targeting agent to a motor neuron comprising the antibody according to the present invention capable of binding to the intravesicular domain (N-terminal portion) of Synaptotagmin 2 (referred to as the "targeting agent according to the present invention").

As used herein, "targeting agent" refers to a drug for delivering a particular substance to a target. In the present specification, by using the targeting agent, a label substance and/or physiologically active substance (referred to as "desired substance") is transported to a motor neuron, in particular, a motor neuron synapse. In addition, in targeting a synapse and a synaptic vesicle, delivery to the cell body is achieved through incorporation into a cell, particularly into a synaptic vesicle, as the synaptic vesicle is recovered via endocytosis. Furthermore, the targeting agent according to the present invention allows the transported substance to exert physiological activity in the cytoplasm.

For example, the transported desired substance may penetrate the synaptic vesicle membrane and migrate into the cytoplasm when the targeting agent according to the present invention is incorporated into a synaptic vesicle. Specifically, for example, where a physiologically active substance is used, at least one physiologically active substance in the targeting agent may migrate to the cytoplasm and act on a biological substance in the nucleus, on a desired biological substance in the cytoplasm, or on a biological substance on the cytoplasmic membrane. The biological substance to which a physiologically active substance acts is not particularly limited as long as it can be present on a cell membrane or in a cell, and may be, for example, any of a polymeric compound such as a protein or a nucleic acid, a low molecular weight compound such as a lipid, a sugar, an amino acid, a nucleotide, and the like, and an ion such as a metal ion, an atom, or the like.

Specifically, for example, a biological substance in the nucleus includes DNA, RNA (such as mRNA, siRNA, miRNA), transcription factor, nuclear receptor, and the like, and a biological substance in the cytoplasm includes, in addition to said nucleic acids, cytoskeleton, enzyme, metal ion, and the like. For example, a biological substance on the cell membrane includes lipid of the cell membrane, receptor on the cell membrane, enzyme coupled with the receptor, cell adhesion factor, and the like.

As used herein, "motor neuron" refers to a group of neurons that transmit central stimuli to the effector, a skeletal muscle. Although a motor neuron generally includes a primary motor neuron that is a central neuron and a secondary motor neuron that is a peripheral neuron, the motor neuron of the present specification is a secondary motor neuron.

As used herein, "secondary motor neuron" refers to a motor neuron that has a cell body in the anterior horn of the spinal cord or brain stem and projects the axon to the junction with skeletal muscles. For example, a motor neuron in the present specification includes α motor neuron, β motor neuron, γ motor neuron, and the like. In addition to spinal neurons having a cell body in the anterior nucleus of the spinal cord, some cranial nerves, such as the oculomotor nerve, trochlear nerve, abducens nerve, facial nerve, and sublingual nerve, are also included. As used herein, a motor neuron normally secretes acetylcholine as a neurotransmitter and is classified as a cholinergic neuron. However, a motor neuron that secretes a neurotransmitter other than acetylcholine may also be used. The muscle cell projected by the motor neuron herein is a skeletal muscle cell.

As used herein, "skeletal muscle cell" refers to a cell constituting a striated muscle that moves the skeleton or a cell having this phenotype. Skeletal muscle cell herein broadly includes a muscle cell attached to bone and other muscle cells comprised in skeletal muscle, such as a muscle spindle. The type of skeletal muscle is not particularly limited, and examples thereof include the diaphragm muscle, vastus lateralis muscle, vastus medialis muscle, rectus femoris muscle, vastus intermedius muscle, biceps brachii muscle, tibialis anterior muscle, tibialis posterior muscle, gastrocnemius muscle, soleus muscle, deltoid muscle, latissimus dorsi muscle, sternocleidomastoid muscle, intercostal muscle, ocular muscle, facial muscle, tongue muscle, stapedial muscle, and the like. The skeletal muscle cell in the present specification also includes a cultured skeletal muscle cell, such as a cell differentiated in vitro from an artificial stem cell (such as an iPS cell and an ES cell) and/or a natural stem cell (such as a mesenchymal stem cell and a skeletal muscle stem cell).

As used herein, a cell may be a cell derived from a vertebrate. Vertebrates include fish, reptiles, amphibians, birds, and mammals. Specific mammals include, for example, primates (e.g., humans). The cell may also be a cell derived from domestic animals (such as chickens, horses, cows, sheep, goats, pigs), pet animals (such as tropical fish, lizards, dogs, cats, rabbits), laboratory animals (such as frogs, mice, rats, monkeys). The cell may not be derived from a single tissue, individual, or animal species, and may be a mixture of multiple types of cells. In addition, the health condition of the tissue and the individual from which the cell is derived is not particularly limited.

According to the targeting agent according to the present invention, the desired substance can be targeted to a motor neuron (e.g., motor neuron axon terminal, axon, axon hillock, cell body, dendrite, and the like) via a motor neuron synapse.

As used herein, "synapse" refers to a junction comprising a cleft formed between an axon terminal of a neuron and a dendrite of another neuron (in the case of the central nervous system) or a cell of skeletal muscle, organ, or the like (in the case of the peripheral nervous system).

As used herein, synapse may be a chemical synapse, such as an excitatory synapse, an inhibitory synapse, or the like. As used herein, synapse may be a synapse formed between a neuron and another neuron (e.g., a synapse formed between an axon of a neuron and a dendrite of another neuron) or a synapse formed between a neuron and another type of cell (e.g., a muscle cell), but is preferably a synapse formed by the presynapse of a neuron and the postsynapse on a skeletal muscle cell (also referred to as a "neuromuscular junction").

As used herein, "presynapse (presynaptic region)" refers to the ampullar portion in a synapse, which is formed at the axon terminal of a neuron, and "postsynapse (postsynaptic region)" refers to the portion of another cell, such as the dendrite of another neuron or skeletal muscle, organ, or the like, that faces the presynapse. In addition, the "synaptic cleft" refers to a space between the presynapse and postsynapse. In a synapse, signals are transmitted by binding to receptors located in the postsynapse by the release of neurotransmitters accumulated in a synaptic vesicle present in the presynapse into the synaptic cleft.

As used herein, "synaptic vesicle" refers to secretory vesicles present in the cytoplasm of the neuron for the presynapse. Synaptic vesicle herein includes not only vesicles that contain a neurotransmitter therein and fuse with the cell membrane in response to a stimulus to release the neurotransmitter into the synaptic cleft, but also vesicles that are recovered into the neurons by endocytosis (including bulk endocytosis) after release of the neurotransmitter.

The targeting agent according to the present invention binds to the intravesicular domain of Synaptotagmin 2 exposed on the cell membrane in the synaptic cleft, and is incorporated into the synaptic vesicle by endocytosis so that it can be delivered to the cell body or the like of the motor neuron. Thus, with the targeting agent according to the present invention, the desired substance (label substance and/or physiologically active substance) can be targeted to the interior of a motor neuron, in particular to the cell body of a motor neuron.

The targeting agent according to the present invention may further comprise a desired substance (label substance and/or physiologically active substance) in addition to an antibody according to the present invention capable of binding to the intravesicular domain (N-terminal portion) of Synaptotagmin 2.

As used herein, "label substance" refers to an agent that emits a detectable signal indicating its presence. Label substance includes, for example, a luminescent label substance that emits light under a certain condition, such as a fluorescent molecule and a chemiluminescent substance, an acoustic label substance that emits sound waves, such as a photoacoustic effect probe, a radioactive label substance, and the like. Examples of fluorescent molecules include, but are not specifically limited to, a fluorescent molecule such as a fluorescent protein, fluorescein and a derivative thereof, pyrene and a derivative thereof, a quantum dot, and the like. The chemiluminescent substance includes, for example, an enzyme such as peroxidase (HRP) and alkaline phosphatase (ALP). The radioactive label substance includes, for example, a reagent including ¹⁴C, ³H, ¹²⁵I, and the like. The photoacoustic effect refers to a phenomenon in which a thermal elastic wave is generated due to adiabatic expansion caused by light absorption, and this thermal elastic wave can be detected as an acoustic wave. The photoacoustic effect probe includes, for example, indocyanine green or a derivative thereof, curcumin derivative, choline derivative, and the like. If the light absorption properties of the antibody, label substance, and physiologically active substance used are known, the label substance for the photoacoustic effect may not necessarily be used, and, for example, the luminescent label substance may be detected on the basis of the photoacoustic effect.

As used herein, "physiologically active substance" refers to a substance capable of exerting a physiological effect directly or indirectly on an organism or a cell. The examples thereof include a low-molecular-weight compound, a functional middle molecule such as a peptide and an aptamer, and a polymeric compound including a biopolymer such as a protein, such as an antibody and an enzyme, and a nucleic acid such as DNA and RNA, which is capable of exerting physiological effects on the target motor neuron. For example, a drug or prodrug such as a synaptogenesis-promoting agent, a synapse-maintaining agent, a muscle-enhancing agent or a neuronal function-modifying agent may be used as a physiologically active substance.

"Physiological effect" refers to an effect that results in a quantitative and/or qualitative change in a biomolecule such as a protein, DNA, or RNA. As a result of the physiological effect, for example, a function and a property of a living body, an organ, a tissue, a cell, and the like may be changed. For example, an effect such as promoting or suppressing synaptogenesis, improving or preventing an impairment in neural function, or improving or preventing an overactivity of neurons may be provided.

Where the targeting agent according to the present invention comprises a desired substance (label substance and/or physiologically active substance), the desired substance is comprised in a state of being not covalently linked (e.g., non-covalently linked) or covalently linked to the antibody according to the present invention. When comprised in a state of being covalently linked, the desired substance and the antibody according to the present invention capable of binding to the intravesicular domain of Synaptotagmin 2, form a conjugate (referred to as "conjugate according to the present invention").

As used herein, "conjugate" refers to a substance in which two or more molecules are covalently linked. In particular, in the conjugate according to the present invention, an antibody according to the present invention and a desired substance (label substance and/or physiologically active substance) are linked.

The covalent and non-covalent bonds between the antibody and desired substance in the targeting agent according to the present invention are not particularly limited as long as it is a bond with which the antibody according to the present invention can reach the vicinity of the motor neuron in a state of being linked to the desired substance.

As used herein, "synaptogenesis-promoting agent" refers to a drug that has the function of promoting the formation of the presynapse and/or postsynapse. Synaptogenesis promotion includes, for example, enhancing the binding strength of the synapse: for example, (i) increasing the surface area and/or volume of the presynapse and/or postsynapse; (ii) increasing and/or qualitatively changing the amount, density, accumulation speed, accumulation frequency, or the like of proteins specifically expressed in the presynapse (e.g., Synapsin 1 or Synapsin 2, or the like); and (iii) increasing and/or qualitatively changing the amount, density, accumulation speed, accumulation frequency, or the like of proteins specifically expressed in the postsynapse (e.g., LRRTM family proteins).

As used herein, "synapse-maintaining agent" refers to a drug that has the function of suppressing the regression of the presynapse and/or postsynapse or assisting the function. Synapse maintenance includes, for example, suppressing the attenuation of the adhesion of synapse or assisting them: for example, (i) suppressing the decrease of the surface area and/or volume of presynapse and/or postsynapse or assisting them; (ii) suppressing the decrease and/or qualitative changes in the amount, density, accumulation speed, accumulation frequency, or the like of proteins specifically expressed in the presynapse (e.g., synapsin 1 or synapsin 2) or assisting them; and (iii) suppressing the decrease and/or qualitative changes in the amount, density, accumulation speed, accumulation frequency, or the like of proteins specifically expressed in the postsynapse (e.g., LRRTM family proteins) or assisting them.

As used herein, "muscle-enhancing agent" refers to a drug that has the function of suppressing the attenuation of or enhancing muscle, or promoting such function. The type of muscle enhancement is not particularly limited as long as the function of the muscle is strengthened and may include: for example, the function of increasing the surface area and/or volume of the muscle; the function of increasing and/or qualitatively changing the density, the number, or the like of each element constituting the muscle, such as the muscle bundle, the muscle fiber, the myofibrils, the sarcomere, the muscle cells, and the like and/or causing a change in the expression level of a specific protein in the cells constituting the muscle; the function of increasing the muscle mass and/or the muscle strength (for example, the muscle mass or the muscle strength of the skeletal muscle); or the function of suppressing the attenuation of the muscle with these functions.

Specific synaptogenesis-promoting agent, synapse-maintaining agent, and muscle-enhancing agent include, but are not limited to, for example, the compounds disclosed in JP 2022-053535 A (e.g., thiamine and derivatives thereof) and the compounds disclosed in JP 2023-028848 A (e.g., atropine, busulfan, chromocarb, procaineamide, udenafil, propifenazone, and derivatives thereof) found by the present inventors.

As used herein, "neuronal function-modifying agent" refers to a drug having the function of altering or promoting a function exerted by a neuron. Functional alteration of a neuron is not particularly limited as long as the degree and/or nature of the function of the neuron changes and includes, for example, changes in the electrophysiological properties of the neuron (such as the properties of conduction and transmission of stimuli), changes in the mode of gene expression, and changes in morphological properties (such as the elongation, regression, and branching of neurite, and the formation and regression of synapse).

Specific functional modifier includes, but are not limited to, for example, AP-1 inhibitor (such as compounds disclosed in WO2020/196725 found by the present inventors), FUS inhibitor, SOD1 inhibitor, TDP-43 inhibitor (e.g., anacardic acid compounds and the like), KIF1A inhibitor, and cytoskeleton-modifying agent such as other microtubule polymerization inhibitors (including auristatin drugs such as monomethyl auristatin E (MMAE), monomethyl auristatin F, and auristatin PE).

As used herein, "cytoskeleton-modifying agent" refers to a drug that suppresses and/or promotes one or more selected from the group consisting of formation, maintenance, degradation, branching, organization, and localization of the cytoskeleton. The cytoskeleton-modifying agent in the present specification also includes an agent that modifies the formation of a cytoskeleton or the like by acting on a molecule other than the cytoskeleton. The cytoskeleton includes any of the microtubules, intermediate filaments, and actin filaments. For example, as the cytoskeleton-modifying agent, an agent that suppresses the formation and maintenance of the cytoskeleton, specifically, for example, an inhibitor of microtubule polymerization or the like, may be used.

These agents may have a function on a neuron and may not have a motor neuron-specific function.

In the conjugate according to the present invention, the antibody according to the present invention and the desired substance (label substance and/or physiologically active substance) may be directly covalently linked, or they may be indirectly linked via a linker or the like.

Where the targeting agent according to the present invention does not comprise the conjugate, it is preferred that the desired substance have a portion capable of binding to the antibody according to the present invention. Specifically, for example, the desired substance covalently linked to another antibody capable of binding to the antibody according to the present invention may be used. A specific configuration of "another antibody capable of binding to the antibody according to the present invention" herein is in accordance with the description of the definitions of" < Antibody According to the Present Invention >", except that the antigen thereof is the antibody according to the present invention, and another antibody may be a polyclonal antibody.

The linkage between the moiety capable of binding to the antibody according to the present invention and the desired substance is in accordance with the linkage in the targeting agent or conjugate according to the present invention. Thus, the moiety capable of binding to the antibody according to the present invention and the desired substance may be non-covalently linked, directly covalently linked, or indirectly linked via a linker or the like.

Where the targeting agent according to the present invention comprises the desired substance, the desired substance may be comprised in the targeting agent according to the present invention in the form of a peptide complex in which the antibody according to the present invention is non-covalently linked to the desired substance.

The site at which the desired substance binds to the antibody according to the present invention is not particularly limited as long as it does not interfere with the binding of the antibody according to the present invention to the SYT2 N-terminus. Specifically, for example, the desired substance may be bound to the constant region or a site other than the hypervariable region (HVR).

In addition, the targeting agent according to the present invention may comprise multiple types of desired substances as long as they do not interfere with each other's function. In this case, for example, multiple identical substances may be comprised, and one or multiple of each of the different substances may be comprised.

As the linker that may be used herein, a linker suitably used in the art may be appropriately employed. The structure and the chain length of the linker herein may be appropriately selected as long as they do not interfere with the function of the resulting conjugate. The linker may, for example, be configured to be cleavable after being transported to the synapse. The linker may also be configured, for example, not to be cleaved after being transported to the synapse.

The linker may be any one commonly used in the art, and is not particularly limited, but, for example, a peptide linker composed of 5 to 25, preferably 10 to 20 amino acid residues, such as a GS linker, may be suitably used. A cleavable linker, for example, an acid-labile linker, a photolabile linker, a peptidase-sensitive linker, a dimethyl linker, a disulfide-containing linker, or the like, may also be used.

The antibody according to the present invention comprised in the targeting agent according to the present invention binds to the intravesicular domain of Synaptotagmin 2 transiently exposed to the cell surface by fusion of a synaptic vesicle with the cell membrane, and can be delivered intracellularly along with Synaptotagmin 2 via endocytosis of the synaptic vesicle. Thus, the targeting agent or conjugate is preferably designed to deliver the desired substance to the synaptic vesicle of the target synapse. The characteristics of compounds that can be delivered to the synaptic vesicle are well known in the art. Since the diameter of the endosome of the bulk endocytosis is 90nm-160nm, the particle diameter of the targeting agent or conjugate according to the present invention may have a mean (or a median) of, for example, 160nm or less, 150nm or less, 140nm or less, 130nm or less, 120nm or less, 110nm or less, 100nm or less, or 90nm or less. Additionally, since the diameter of the synaptic vesicle is 40nm-60nm, the particle diameter of the conjugate according to the present invention may have a mean (or a median) of, for example, 60nm or less, 55nm or less, 50nm or less, 45nm or less, 40nm or less, 35nm or less, 30nm or less, 25nm or less, 23nm or less, 20nm or less, 18nm or less, 15nm or less, 14nm or less, 13nm or less, 12nm or less. For example, if the targeting agent itself does not comprise the desired substance, the entire particle diameter when the desired substance is bonded to the targeting agent may be within the above-described ranges. However, the particle diameter may be designed to be large for the purpose of, for example, inhibiting endocytosis of the synaptic vesicle or delivering a substance to the surface of the synapse or synaptic cleft. Further, for example, if the desired substance is designed to be already separated when delivery to the synaptic vesicle is made, the particle diameter prior to separation may be designed to be large.

The conjugate or targeting agent according to the present invention may not be configured to cross the blood-brain barrier. Normally, the conjugate or targeting agent according to the present invention does not cross the blood-brain barrier and therefore does not act on the central nervous system and can only act on the peripheral synapse.

Targeting function to motor neuron via synapse may be determined, for example, by administering the conjugate or targeting agent according to the present invention, comprising the physiologically active substance, to a subject such as a vertebrate (e.g., a non-human mammal, human, or other vertebrate) and assessing the physiological effects of the conjugate or targeting agent according to the present invention on the neuron of the subject. The physiological effect may be assessed, for example, by comparing the degree of physiological effect between the group receiving the conjugate or targeting agent according to the present invention and the group without receiving it, and/or by comparing the degree of physiological effect between the group receiving the conjugate or targeting agent according to the present invention and the group receiving the physiologically active substance alone.

The present inventors have previously found that presynapse formation can be induced by co-culturing a neuron with microbeads on the surface of which an LRRTM molecule (such as the extracellular domain of LRRTM2) is immobilized (WO2021/006075).

Thus, whether a tested substance has a targeting function on a motor neuron, including the synapse, may also be determined by examining whether the tested substance is localized on the presynapse induced by co-culturing neurons with microbeads.

"LRRTM (leucine-rich repeat transmembrane neuronal protein) family protein" refers to proteins belonging to the LRRTM family. The LRRTM family is one of the synapse organizer protein families on the side of the postsynapse and has the activity of inducing presynapse formation. In mammals, including humans, four types of LRRTM family proteins have been reported: LRRTM1, LRRTM2, LRRTM3, and LRRTM4. LRRTM family protein used for the microbeads may be any of them.

### < Conjugate >

The present invention relates to a conjugate of the antibody according to the present invention capable of binding to the intravesicular domain (N-terminal portion) of Synaptotagmin 2 and a desired substance (label substance and/or physiologically active substance). The conjugate according to the present invention is, for example, delivered to a motor neuron by incorporation into a synaptic vesicle of the motor neuron.

### < Visualizing Agent of a Motor Neuron or Synapse thereof >

The present invention provides a targeting agent, which is a visualizing agent of a motor neuron or synapse thereof (referred to as a "visualizing agent according to the present invention").

The visualizing agent according to the present invention is a targeting agent comprising a label substance for use in visualizing a motor neuron or a synapse thereof.

"Visualizing motor neuron" refers to making all or part of a motor neuron in a detectable state. When visualizing a part of a motor neuron, the part to be visualized may be a random or predetermined part. For example, a synapse may be visualized as a predetermined part. The visualizing agent according to the present invention can then be used as a synapse visualizing agent. "Visualizing synapse" refers to making presynapse and/or postsynapse in a detectable state. Therefore, any detectable label substance, as well as the label substance that can be directly detected by visual observation, may be used in the visualizing agent according to the present invention. Similarly, the visualizing agent according to the present invention may visualize an axon terminal, an axon, an axon hillock, a cell body, a dendrite, and the like of a motor neuron.

The visualizing agent according to the present invention may be used in vivo or in vitro. The signal of the label substance may be detected while the motor neuron is alive or after the motor neuron is fixed. The label substance suitable for detecting a motor neuron in a living condition are known in the art. For example, a fluorescent substance known in the field of in vivo imaging, a luminescent substance such as a chemical or bioluminescent substance, a sound-emitting substance such as a photoacoustic probe, a radioactive substance such as a radioisotope, a contrasting agent, and the like may be included.

The visualizing agent according to the present invention may be used for any application, for example, to visualize the number, size, or position of motor neurons, synapses (including neuromuscular junctions), or synaptic vesicles, or to visualize tissue in a surgical or diagnostic procedure.

The visualizing agent according to the present invention may be provided in the form of a kit together with, for example, other reagents, such as reagents required for detecting the label substance comprised in the visualizing agent according to the present invention.

In particular, if, for example, the label substance is an enzyme or the like, the substrate may be provided with the visualizing agent according to the present invention.

### < Composition or Pharmaceutical Composition >

The present invention further relates to a composition (referred to as the "composition according to the present invention") or a pharmaceutical composition (referred to as the "pharmaceutical composition according to the present invention") comprising a conjugate or a targeting agent according to the present invention.

A composition or a pharmaceutical composition according to the present invention comprises the targeting agent according to the present invention comprising a physiologically active substance. In addition to the targeting agent, the composition or pharmaceutical composition according to the present invention may optionally comprise an additive (such as a carrier (a solid, liquid carrier, and the like), an excipient, a surfactant, a binder, a disintegrant, a lubricant, a solubilizing agent, a suspending agent, a coating agent, a colorant, a preservative, a buffer, a pH modifier), and the like. The additive herein may be appropriately selected according to the formulation or dosage form of the composition or pharmaceutical composition.

The composition or pharmaceutical composition according to the present invention may be prepared in any dosage form, such as, but not limited to, a solid formulation, a liquid formulation, a gel, an aerosol, or the like. If the composition or pharmaceutical composition is used as a liquid formulation, it may also be prepared as a dry product intended for reconstitution with, for example, saline immediately prior to its use.

The excipient includes, for example, lactose, crystalline cellulose, starch, and the like. The binder includes, for example, starch paste, gum arabic paste, hydroxypropylcellulose, and the like. The disintegrant includes, for example, starch, celluloses, carbonates, and the like. The lubricant includes, for example, wax, talc, and the like.

When the composition or pharmaceutical compositions according to the present invention comprising the synaptogenesis-promoting agent, the synapse-maintaining agent, the muscle-enhancing agent, or the neuronal function-modifying agent as a physiologically active substance, the impairment in neural function can be improved or prevented by promoting synaptogenesis. Therefore, the composition or pharmaceutical composition according to the present invention may be used to ameliorate or prevent an impaired neural function, for example, an impairment in neural function due to nerve injury, an impairment in neural function due to aging, an impairment in neural function due to diseases or the like, or to improve neural function.

As used herein, "nerve damage" refers to damage at any point in the nerve, including damage physically caused from outside the body and damage caused by factors in the body such as cancer, tumors, and the like.

As used herein, "aging" refers to various functional impairments, changes in morphology, changes in appearance, and the like that occur in an individual organism over time, and the processes thereof.

Frail, sarcopenia, and the like are known as conditions caused by aging. "Frail" refers to a condition in which mental and physical vitality (motor function, cognitive function, or the like) are reduced with aging, impairments in living functions, and mental and physical weakness occur, while being affected by the coexistence of multiple chronic diseases, or the like. The reduction in mental and physical vitality includes, for example, cognitive dysfunction, dizziness, eating disorders, dysphagia, visual impairment, depression, anemia, hearing loss, delirium, compromised infectivity, weight loss, muscle mass loss, and the like. The chronic disease includes hypertension, heart disease, cerebrovascular disease, diabetes, respiratory disease, malignancy, and the like. On the other hand, sarcopenia means a condition in which muscle strength is reduced while skeletal muscle mass is reduced due to aging, disease, or the like. In the neuromuscular junction of aged mice, morphological changes such as synaptic detachment and partial or complete axonal detachment from the postsynapse are observed, and thus, it is believed that changes in the morphology of the neuromuscular junction with aging are involved in the reduction of skeletal muscle mass and the like in sarcopenia.

The composition or pharmaceutical composition according to the present invention may be used to ameliorate or prevent an impairment in neural function due to aging, particularly in a subject having or at increased risk of having frail or sarcopenia.

In the present invention, disease includes, for example, neurological disease and neuromuscular disease.

As used herein, "neurological disease" refers to a disease caused by a disorder of a nerve, such as a central nerve or a peripheral nerve, and refers to, for example, one or more diseases selected from the group consisting of Alzheimer's disease, Parkinson's disease, dementia of Lewy bodies, frontotemporal lobar degeneration, progressive supranuclear palsy, corticobasal degeneration, Huntington's disease, dystonia, prion disease, chorea-acanthocytosis, adrenoleukodystrophy, multiple system atrophy, spinocerebellar degeneration, amyotrophic lateral sclerosis, primary lateral sclerosis, bulbar spinal muscular atrophy, spinal muscular atrophy, spastic paraplegia, syringomyelia, Charcot-Marie-Tooth disease, frontotemporal dementia, epilepsy, schizophrenia, autism, autism spectrum disorder, or the like.

As used herein, "neuromuscular disease" refers to a disease caused by a disorder of any of the motor nerves, neuromuscular junctions, or muscle cells, for example, one or more diseases selected from the group consisting of muscular dystrophy, myopathy, congenital myasthenia syndrome, hereditary periodic quadriplegia, myasthenia gravis, Lambert-Eaton syndrome, and the like.

Amyotrophic lateral sclerosis (ALS) is a disease in which the primary motor nerve and the secondary motor nerve are selectively and progressively degenerated and disappear, and it is known that the motor nerve is detached from the skeletal muscle at the neuromuscular junction as an initial pathology. Thus, the composition or pharmaceutical composition according to the present invention comprising the synaptogenesis-promoting agent or synapse-maintaining agent capable of promoting the formation of synapses between skeletal muscle and motor nerve or suppressing the regression may be used to ameliorate or prevent, in particular, an impairment in neural function due to amyotrophic lateral sclerosis.

For example, the pharmaceutical composition according to the present invention may be a pharmaceutical composition for the treatment of amyotrophic lateral sclerosis, a pharmaceutical composition for the treatment of spinal muscular atrophy, or the like, comprising the conjugate or targeting agent according to the present invention.

A composition or pharmaceutical composition according to the present invention comprising the muscle-enhancing agent may improve or prevent muscle weakness by augmenting muscle. Thus, the composition or pharmaceutical composition according to the present invention comprising the muscle-enhancing agent may be used to ameliorate or prevent muscle weakness, such as muscle weakness following trauma or surgery, muscle weakness due to aging, muscle weakness due to diseases, or to improve muscle function.

As used herein, "trauma" refers to damage to tissues or organs caused by external factors, including, for example, wounds, fractures, sprains, visceral ruptures, burns, frostbites, and the like.

The composition or pharmaceutical composition according to the present invention comprising the function-modifying agent may improve or prevent an impairment or an increase in neurons by altering the function of a motor neuron. Thus, the composition or pharmaceutical composition according to the present invention comprising the function-modifying agent can also be used to ameliorate or prevent neuronal overactivity, such as neuronal overactivity due to a disease or condition, or to ameliorate or prevent muscle tone, such as muscle tremor due to aging or muscle tone due to trauma or disease.

Specifically, it may be used to improve or prevent various diseases and conditions described above, including abnormal involuntary movements (dyskinesias) (e.g., abnormal head movements, tremors, (painful) convulsions, muscle fasciculations), abnormal gait and movement (e.g., atactic gait, difficulty walking), other coordination disorders (e.g., ataxia), other conditions related to the nervous system and musculoskeletal system (e.g., tetany, abnormal reflexes, postural abnormalities, spasticity, muscle hypertonia, muscle tone, exaggerated deep tendon reflexes, dysphagia), and the like.

As used herein, "disease" refers to a pathological condition that can be classified by identifiable symptoms or causes in a subject individual and includes illness and disorders. In the present invention, "condition" refers to a pathological condition including a discernible symptom in a subject individual that is not classified as a disease.

The composition or pharmaceutical composition according to the present invention may comprise multiple targeting agents according to the present invention and may further contain another active ingredient. Another active ingredient is not particularly limited as long as it does not interfere with the function of the targeting agent comprised in the composition or pharmaceutical composition. Where multiple types of targeting agents are comprised, a targeting agent capable of binding to a membrane protein other than Synaptotagmin 2 may be comprised. Additionally, for example, the targeting agents comprising the antibody capable of binding to distinct sites (e.g., distinct epitopes) in Synaptotagmin 2 may be comprised. The label substance and/or physiologically active substance comprised in these targeting agents may be different or the same from each other.

The nucleic acid molecule and/or cell according to the present invention may be comprised in the composition or pharmaceutical composition according to the present invention.

### < Method for Targeting to a Motor Neuron or Synapse >

The present invention relates to a method for targeting to a motor neuron or synapse. According to the present invention, a desired substance (label substance and/or physiologically active substance) can be targeted to a motor neuron (e.g., an axon terminal, an axon, an axon hillock, a cell body, a dendrite, and the like of motor neuron) by contacting a motor neuron with the antibody according to the present invention that binds to the intravesicular domain of Synaptotagmin 2, and allowing the antibody to be incorporated into motor neuron (within synaptic vesicle of motor neuron, in particular). In this way, the antibody that binds to the intravesicular domain of Synaptotagmin 2 is targeted to the motor neuron or synapse. The desired substance can be delivered within a motor neuron (e.g., within a cell body or synaptic vesicle of the motor neuron) by directly or indirectly linking the desired substance to the antibody via a linker to produce the conjugate according to the present invention and contacting the conjugate with the motor neuron, or by separately contacting the antibody and desired substance capable of binding to the antibody with the motor neuron. Thus, according to the present invention, there is provided a method for targeting to a motor neuron or synapse, comprising contacting cells, such as the motor neuron, with an antibody (which may be linked to a desired substance).

### < Method for Targeting the Label Substance and/or Physiologically Active Substance >

The present invention relates to a method for targeting a desired substance (label substance and/or physiologically active substance) comprising a step of contacting a conjugate or a targeting agent according to the present invention with a motor neuron and a step of delivering the targeting agent to the motor neuron synapse.

The subject for the contact in the present invention is not particularly limited as long as it comprises a motor neuron. The contact may be performed using, for example, only a motor neuron alone as a subject or a tissue comprising cells other than the motor neuron as a subject. For example, because the targeting agent according to the present invention primarily targets the presynaptic region of motor neuron synapse at the neuromuscular junction, it may be directed to a tissue further comprising skeletal muscle cells projected by a motor neuron.

Motor neuron in the present method may comprise a motor neuron of a vertebrate (non-human mammal, human, or other vertebrate). Motor neuron in the present method preferably comprises a human motor neuron. Hereinafter, the present invention will be described with reference to a human motor neuron, but the present method is not limited to that of a human motor neuron.

The human motor neuron may be used without limitation regardless of its origin. Examples include, but are not limited to, a primary culture of a cell isolated from humans, a cell isolated from humans and established as a cell line, and a human motor neuron into which a pluripotent stem cell derived from humans is induced to differentiate. The pluripotent stem cell from which the human motor neuron is derived is preferably a pluripotent stem cell from a human.

In one embodiment, the present method is a method of in vitro. In another embodiment, the present method is a method of ex vivo. In another embodiment, the present method is a method of in vivo. Where the present method is a method of in vivo, the subject may be a mammal other than a human.

Where the present method is a method of in vitro, the present method may further comprise the step of inducing synaptogenesis. As used herein, "inducing synaptogenesis" refers to causing the formation of the presynapse in the axon of a neuron and/or causing the formation of the postsynapse at a dendrite of another neuron, or at a cell, such as of skeletal muscles or organs. Synaptogenesis may be induced by co-culturing a cell to form a presynapse and a cell to form a postsynapse. Synaptogenesis may also be induced by other methods, for example, by co-culturing a motor neuron with beads coated with the extracellular domain of LRRTM2. The step of inducing synaptogenesis may be performed simultaneously with or prior to the step of contacting the targeting agent according to the present invention with a motor neuron.

Where the present method is a method of in vitro, the step of contacting the targeting agent according to the present invention with a motor neuron is performed by contacting the targeting agent according to the present invention with a sample containing a motor neuron. The method of contacting is not particularly limited as long as a motor neuron in the sample and the targeting agent may be brought into contact with each other. For example, targeting agent may be applied to the sample by directly sprinkling, spraying, dropping, spreading, immersing the sample in targeting agent, or a combination thereof. In addition, where the sample is present in another carrier (for example, a culture medium or the like), the targeting agent may be applied to the carrier by sprinkling, spraying, dropping, or spreading.

The amount to be applied is not particularly limited and may be appropriately set considering the number of motor neurons and other conditions. The application may be performed at a concentration of, for example, 0.01µg/mL or more, 0.1µg/mL or more, 0.2µg/mL or more, 0.5µg/mL or more, 0.7µg/mL or more, 0.9µg/mL or more, 1µg/mL or more, 2µg/mL or more, 5µg/mL or more, 7µg/mL or more, 9µg/mL or more, or 10µg/mL or more, as a concentration of the IgG antibody.

Where the present method is a method of in vivo, the step of contacting the targeting agent according to the present invention with the test sample is performed by administering the targeting agent according to the present invention to the subject.

Examples of the administration method include, but are not particularly limited to, topical administration, enteral administration, and parenteral administration, specifically, dermal administration, inhalation administration, enema administration, eye drops, ear drops, nasal administration, intravaginal administration, administration through tube feeding, intravenous administration, intraarterial administration, intramuscular administration, intracardiac administration, subcutaneous administration, intraosseous administration, intradermal administration, intrathecal administration, intraperitoneal administration, intravesical administration, transdermal administration, transmucosal administration, epidural administration, intravitreal administration, and the like.

The dose is not particularly limited and may be appropriately set considering the animal species of the subject and other conditions. For example, where IgG antibody is administered to mice, the dose per 1kg of body weight may be 0.1mg/kg or more, 0.5mg/kg or more, 1mg/kg or more, 2mg/kg or more, 4mg/kg or more, or 5mg/kg or more.

The targeting agent used in this step may not be of a single type. For example, multiple types of targeting agents may be used together or separately. Specifically, for example, the targeting agent comprising a conjugate and the targeting agent not comprising a desired substance (label substance and/or physiologically active substance) may be used. Additionally, a desired substance used may not be one type, and for example, multiple types of label substance and/or physiologically active substance may be used together or separately. Specifically, for example, a label substance and a physiologically active substance may be used in combination.

The step of contacting the targeting agent according to the present invention with a motor neuron may be performed multiple times. Where this step is performed multiple times, the types of the targeting agent and cell, as well as the method of application and administration used in each performance, may be the same or different each time.

Upon contacting the motor neuron with the conjugate or targeting agent according to the present invention, the targeting agent according to the present invention is incorporated into the motor nerve via a synaptic vesicle. When the targeting agent according to the present invention is contacted with the motor neuron, the motor neuron may be activated, or the activity thereof may be promoted. By activating or promoting the activity of the motor neuron, the conjugate or targeting agent according to the present invention can be more efficiently incorporated into the motor neuron. Usually, when the conjugate or targeting agent according to the present invention is incorporated into a synaptic vesicle, it is delivered to the cell body through axons by retrograde transport.

The methods of activating a motor neuron include, but are not particularly limited to, for example, the method to allow spontaneous activity of the motor neuron over a sufficient period of time and the method of activating a motor neuron or promoting endocytosis of a synaptic vesicle by artificial stimuli.

Methods to allow spontaneous activity include, for example, placing a motor neuron in an environment to allow activity over a sufficient period of time. The environments to allow a motor neuron to be active are well known in the art.

Time period (duration) for activity in the method to allow spontaneous activity of a motor neuron (e.g., the time period of contacting with the conjugate or targeting agent according to the present invention) is not particularly limited but may be, for example, where the present method is an in vitro method, 1 hour or more, 3 hours or more, 6 hours or more, 12 hours or more, 18 hours or more, or 24 hours or more. Where the present method is an in vivo method, it may be, for example, 1 hour or more, 3 hours or more, 6 hours or more, 12 hours or more, 18 hours or more, 24 hours or more, 36 hours or more, 48 hours or more, 60 hours or more, 72 hours or more, 100 hours or more, 120 hours or more, 150 hours or more, 168 hours or more, 200 hours or more, or 240 hours or more.

Methods of activating or promoting the activity of a motor neuron by artificial stimuli include, for example, placing the motor neuron in an environment where a motor neuron can be highly active for a sufficient period of time.

Where the present method is an in vitro method, a chemical stimulus and/or a physical stimulus can be provided to the motor neuron in the method of activating or promoting the activity of the motor neuron. Stimuli for strong activation of a motor neuron are well known in the art. Compounds used for chemical stimulation include, for example, a potassium ion channel inhibitor such as amiodarone, tetraethylammonium, 4-aminopyridine, barium, dendrotoxin, a sodium channel agonist such as batracotoxin, a calcium channel agonist such as Bay K8644, high concentration of potassium ions or neurotransmitters, or combinations thereof. Examples of physical stimuli include a temperature change.

When chemical stimulation is performed, the amount of the compound added is not particularly limited. For example, addition may be at a concentration of 1µM or more, 10µM or more, 50µM or more, or 100µM or more.

The duration of the stimulation is not particularly limited and may be appropriately set considering conditions such as the type and intensity of the stimulation. For example, stimulus may be applied for 2 minutes or more, 3 minutes or more, 4 minutes or more, 5 minutes or more, 8 minutes or more, 9 minutes or more, 10 minutes or more, 20 minutes or more, 25 minutes or more, 30 minutes or more, or 1 hour or more.

Where the present method is an in vivo method, the method of activating or promoting the activity of a motor neuron may be performed by, such as, a method to allow the subject to be highly active (e.g., a method to allow the subject to have exercise or a method of activating the brain activity), a method of promoting the activity of the motor neuron by a chemical or the like. Compounds that promote motor nerve activity include those compounds used for the chemical stimulation described above and may be administered to a subject. The compound used for the chemical stimulus may be administered at a pharmaceutically acceptable concentration or method. The compound may be administered to a subject such that the compound stimulus does not exhibit biotoxicity, but the compound may not be administered to the subject if the biotoxicity is manifested. Promoting motor neuron activity is usually expected to provide a comparable effect in a short time compared to the method to allow firing naturally.

Where the targeting agent without a desired substance is used, the desired substance and targeting agent may be contacted with a motor neuron together or separately. If the desired substance and the targeting agent are contacted separately, the timing is not particularly limited as long as the desired substance may be combined with the targeting agent. For example, the contacting of the desired substance with the motor neuron may be started before the contacting of the targeting agent with the motor neuron or thereafter.

The method for each contact may be selected as the method of contacting described above. For example, the same method or different methods may be used for each contact.

The present method may optionally further comprise the step of confirming the success or failure of targeting. If the targeting agent used in the present method comprises a physiologically active substance, targeting may be determined to be successful if, for example, a physiological effect is observed as described above in the targeting agent section. In addition, if the targeting agent used in the present method comprises a label substance, targeting may be determined to be successful when the signal is detected, as in the step of detecting the signal of the label substance in the method for visualizing, which will be described later.

When the pharmaceutical composition according to the present invention is used as a targeting agent for the present method, the present method may be used as a method for preventing or treating a condition or a disease.

In this case, the method may further comprise a step of sufficiently exerting a physiological effect on the subject. For example, when a physiologically active substance to be used is a substance capable of exerting an effect alone, the effect may be exerted by placing the subject in an environment in which nutrients are sufficiently provided for a sufficient period of time for the physiological effect to be exerted. Additionally, for example, if a physiologically active substance to be used requires another substance to exert an effect, the substance may be administered additionally.

The present invention thereby relates to a method for preventing or treating a condition or a disease comprising a step of contacting a targeting agent comprising an antibody according to the present invention and a physiologically active substance with a motor neuron, and a step of delivering said targeting agent to said motor neuron synapse. According to the present method, various conditions or diseases exemplified with respect to the pharmaceutical composition may be prevented or treated. The contacting step of the present method preferably comprises administering the targeting agent and/or pharmaceutical composition to the subject.

Thus, for example, the method according to the present invention is a method for ameliorating or preventing an impairment in neural function, such as an impairment in neural function due to nerve damage, an impairment in neural function due to aging, or an impairment in neural function due to a disease, or for improving neural function. In one embodiment, the condition or disease is a condition or a disease that exhibits impaired neural function. In one embodiment, the condition or disease is a neurological disease and a neuromuscular disease. In one embodiment, the targeting agent comprises a conjugate of the antibody and the physiologically active substance.

The duration of this step may be determined as appropriate depending on the condition of the subject, the type of the physiologically active substance, the dose, and the like. For example, a determination may be made based on the duration generally required for a physiological effect to be exerted when the physiologically active substance is administered, and the physiological effect may be confirmed once or multiple times and continued until the physiological effect is sufficiently exerted.

### < Method for Visualizing a Motor Neuron or Synapse thereof >

The present invention relates to a method for visualizing a motor neuron or synapse comprising a step of contacting a visualizing agent according to the present invention with a motor neuron, a step of delivering the visualizing agent to a motor neuron synapse, and a step of detecting the signal of the label substance.

In the present method, the motor neuron used for contacting is as described for the method for targeting a label substance and/or a physiologically active substance described above.

In one embodiment, the present method is a method of in vitro. In another embodiment, the present method is a method of ex vivo. In another embodiment, the present method is a method of in vivo. Where the present method is an in vivo method, the subject may be a human or a mammal other than a human.

In the present method, the step of contacting the visualizing agent according to the present invention with a motor neuron and the step of delivering the visualizing agent to a motor neuron synapse are the same as the step of contacting the targeting agent according to the present invention with a motor neuron and the step of delivering the targeting agent to a motor neuron synapse described for the method for targeting a label substance and/or a physiologically active substance described above, except that the visualizing agent is used in place of the targeting agent.

The present method may optionally further comprise a step of generating a signal from the label substance. The method of generating the signal is not particularly limited. The method of generating the signal and the necessity thereof may be determined based on the type of label substance to be used or the like.

For example, if the label substance is a fluorescent molecule or a radioactive label substance, a signal may be generated at the target by waiting for a time sufficient for the visualizing agent to be delivered to the target motor neuron synapse, that is, a time sufficient for the visualizing agent to reach the target motor neuron synapse and for endocytosis of a synaptic vesicle to occur at that motor neuron synapse. This time may be appropriately selected as in the time of the step of delivering a visualizing agent to a motor neuron synapse.

For example, if a label substance is a chemiluminescent material, it may be done by adding a substance, such as its substrate used to generate the signal, in addition to waiting for a sufficient time for the visualizing agent to be delivered to the target motor neuron synapse.

This step may be performed at the same time as or before the step of detecting a signal described below.

The present method further comprises the step of detecting the signal of the agent for detection. The method used for detecting is not particularly limited and may be appropriately selected according to conditions such as the type of the label substance to be used.

Where the label substance is a fluorescent material, for example, a motor neuron may be irradiated with excitation light containing light with an excitation wavelength for the label substance, and the detection may be performed using a detector capable of detecting the fluorescence wavelength of the label substance. In addition, where the label substance is a chemiluminescent material, for example, the detection may be performed using a detector capable of detecting the emission wavelength of the label substance. Where the label substance is a radioactive label substance, the detection may be performed using a detector capable of detecting the radiation emitted by the label substance.

Detecting the signal of the label substance includes detecting the presence, location, or quantity of a motor neuron (e.g., synapse, cell body, or the like) in a sample containing a motor neuron.

In addition to the step of detecting the signal of the label substance in the sample, the methods according to the present invention may further comprise the step of comparing the signal of the label substance detected in the sample to a signal in a standard sample comprising the label substance or a pre-established reference value to determine the presence, location, or quantity. The standard sample is not particularly limited as long as it is a biological sample that serves as a reference for determining whether to have a specific condition or disease. Specifically, for example, those obtained from a healthy individual, those obtained from the same individual as the sample at different times of collection, or those obtained from an individual known to have a specific condition or disease. The standard sample may be, for example, a biological sample derived from the same biological species, individual, tissue, or cell as the sample, or may be a biological sample derived from another biological species, individual, tissue, or cell. Further, the reference value is not particularly limited as long as it is a reference value for determining whether to have a condition of interest. The reference value may be set based on, for example, the intensity, number, or the like of signals generally detected in the standard sample.

In any case, the method of comparison is not particularly limited. For example, comparison may be performed by visual observation, by numerical magnitudes, or by a statistical method.

### < Other Inventions >

The present invention provides a method of administering a desired substance to a subject. The substance is in the form of a conjugate of the antibody according to the present invention and the desired substance. The desired substance can thereby be delivered to the cell, such as a motor neuron, of the subject. Where the desired substance is a physiologically active substance, the physiologically active substance can be delivered to a cell, such as a motor neuron. Additionally, where the desired substance is a label substance, it may be used to observe the delivery site of the label substance (e.g., motor neuron or synapse thereof). The present invention also provides a conjugate of the antibody and the desired substance, or a composition comprising the conjugate, for use in this method. The present invention relates to a composition for targeting to a motor neuron or a synaptic thereof comprising the antibody according to the present invention, or a conjugate of the antibody with a desired substance (label substance and/or physiologically active substance).

The present invention provides a method for visualizing a motor neuron in a subject, comprising administering to the subject an effective amount of a conjugate of the antibody according to the present invention and a label substance. The present invention also provides a conjugate of the antibody and the label substance or a composition comprising the conjugate, for use in this method.

The present invention provides a method of delivering a physiologically active substance to a motor neuron of a subject, comprising administering to the subject an effective amount of a conjugate of the antibody according to the present invention and the physiologically active substance. The present invention also provides a conjugate of the antibody and the physiologically active substance or a composition comprising the conjugate, for use in this method.

The present invention provides the antibody according to the present invention or the conjugate according to the present invention for use in any of the above methods. For example, the present invention relates to the antibody according to the present invention or the conjugate according to the present invention for use in a method of preventing or treating a condition or a disease. Further, for example, the present invention relates to the antibody according to the present invention or the conjugate according to the present invention for use in the method for targeting the label substance and/or the physiologically active substance to a motor neuron or a synapse thereof.

The present invention provides the antibody or a conjugate of the antibody and the substance for use in the manufacture of a medicament, for use in any of the methods described above.

The present invention also provides the use of the antibody according to the present invention or the conjugate according to the present invention in the manufacture of a medicament comprising the antibody and the physiologically active substance.

### Examples

Hereinafter, the present invention will be described in more detail with reference to Examples. However, the technical scope of the present invention is not limited to these Examples.

Between the present application and Japanese Patent Application No. 2023-090263, Examples 1 to 4, Figures 1 to 5, Tables 1 to 6, and SEQ ID NOs: 1 to 17 correspond to the same numbers, respectively.

### < Example 1: Production of the First Monoclonal Antibody >

A monoclonal antibody was produced using phage display by commission to Medical & Biological Laboratories Co., Ltd. Production procedures are summarized as follows.

### 1. Synthesis of Antigenic Peptide

The following peptides were synthesized as an antigen for immunization and antigens for pannings by commission to Beijing SciLight Biotechnology Ltd. Co.

**[Table 1]**

| Table 1: Synthesized antigenic peptide | | | | | |
|---|---|---|---|---|---|
| | | Parent protein | Position | Sequence | SEQ ID NO |
| Antigenic peptide for immunization | im-hSYT2 | Human SYT2 | 1-25 | MRNIFKRNQEPIVAPATTTATMPIGC | 14 |
| Antigenic peptide for panning | pan-hSYT2 | Human SYT2 | 1-62 | MRNIFKRNQEPIVAPATTTATMPIGP VDNSTESGGAGESQEDMFAKLKEKLF NEINKIPLPPK* | 15 |
| | pan-mSYT2 | Mouse SYT2 | 1-65 | MRNIFKRNQEPNVAPATTTATMPLAP VAPADNSTESTGPGESQEDMFAKLKE KFFNEINKIPLPPK* | 16 |
| | pan-hSYT1 | Human SYT1 | 1-58 | MVSESHHEALAAPPVTTVATVLPSNA TEPASPGEGKEDAFSKLKEKFMNELH KIPLPPK* | 17 |

| | | | | | |
|---|---|---|---|---|---|
| Underline: cysteine residue for carrier protein binding *: lysine residue bound to biotin | | | | | |

The antigen for immunization was bound to a carrier protein using Ellman's reagent in order to induce an immune response. The carrier protein used was keyhole limpet hemocyanin (KLH).

### 2. Immunization of the Mouse

The KLH-bound antigen for immunization was prepared as a 1.0 mg/mL antigen solution, and four mice were immunized a total of 4 times, once every week. Serum for antibody titer confirmation and lymph node tissues for phage library production were collected from the mice after the completion of immunization.

The obtained serum was evaluated for an antibody titer by ELISA using three types of antigenic peptides for panning synthesized in "1. Synthesis of Antigenic Peptide" as antigens. As a result, an antibody capable of binding to the N-terminal domain of human Synaptotagmin 2 was confirmed to be present in the collected mouse serum.

### 3. Production of Antibody Phage Library

Total RNA was extracted from cells of the lymph node tissues collected from the immunized mice, and reverse-transcribed to prepare cDNA. Next, VH and VL gene regions were amplified from the prepared cDNA using primers for antibody gene amplifications. The amplified nucleic acids were integrated into phagemid vectors to produce an antibody phagemid library. E. coli was transformed using the produced antibody phagemid library to produce an antibody phage library (which would express an antibody fragment scFv and display it on the surface).

### 4. Enrichment by Panning

A series of panning operations shown in the following steps (1) to (3) was repeated 3 times so that phages expressing fragments of the target antigen-specific antibody were enriched:
(1) the antibody phage library was contacted with an excluded antigen (negative antigen), and antibody phages that reacted with the excluded antigen were removed, while unreacted antibody phages were collected;
(2) the collected antibody phages were contacted with a target antigen, and antibody phages that reacted with the target antigen were collected, while unreacted antibody phages were washed off; and
(3) E. coli was infected with the collected antibody phages and allowed to proliferate by culture.

The excluded antigen and the target antigen used in each panning operation are shown in Table 2.

**[Table 2]**

| Table 2: Antigen used in panning operation | | |
|---|---|---|
| | Excluded antigen | Target antigen |
| 1st round | pan-hSYT1 | pan-hSYT2 |
| 2nd round | pan-hSYT1 | pan-mSYT2 |
| 3rd round | HEK293T | HEK293T (hSYT2 expression) |

In the final panning operation, HEK293T cells (manufactured by ATCC; Cat. No: #CRL-3216) and these cells harboring a transient expression vector for human Synaptotagmin 2 (expressed under CMV promoter) were used.

### 5. Evaluation of enrichment based on antibody titer

In each panning operation, the expression of the antibody gene was induced in the E. coli infected in step (3), and the titer of an antibody (polyclonal scFv) in a culture supernatant was evaluated by ELISA using three types of antigenic peptides for panning synthesized in "1. Synthesis of Antigenic Peptide" as antigens. After the final panning operation, the titer of the antibody (polyclonal scFv) was evaluated using the culture supernatant of each of 96 E. coli colonies thus collected. 79 clones were selected as clones that reacted with the N-terminal domain of human and mouse Synaptotagmin 2 and did not react with the N-terminal domain of human Synaptotagmin 1.

### 6. Identification of the Amino Acid Sequence of scFv

The nucleotide sequences of the antibody genes of the selected clones were determined by commission to Azenta Inc., and the amino acid sequence of scFv was identified.

### 7. Screening of Clone

Eight clones for IgG antibody production were screened for on the basis of the results of cluster analysis based on the sequence identity of amino acid sequences and the results of reactivity tests for two types of HEK293T cells used in the panning operations.

### 8. Production of Humanized IgG Antibody

The gene regions of the H chain and L chain variable regions (mVH and mVL, respectively) were amplified by PCR from the scFv expression vector of each of the eight clones screened for. The amplified VH was inserted together with a human IgG1 constant region (hCH) into a pcDNA3.4 plasmid (heavy chain expression vector), and likewise, VL was inserted together with a human Igκ constant region (hCL) into a pcDNA3.4 plasmid (light chain expression vector).

Expi CHO^{™} cells were co-transfected with the heavy chain expression vector and the light chain expression vector, and a culture supernatant was collected.

The antibody was purified from the obtained culture supernatant using Pierce^{™} Disposable Columns, 10 mL (Cat. No: #29924; Thermo Fisher Scientific).

The antibody was thereby produced in an amount of 15 mg or more from each clone.

Of the obtained antibodies, one clone was used as the monoclonal antibody according to the present invention. The amino acid sequences of the variable regions of the monoclonal antibody according to the present invention are shown in Table 3.

**[Table 3]**

| Table 3: Amino acid sequences of variable regions of the monoclonal antibody of the present invention | | |
|---|---|---|
| | Sequence | SEQ ID NO |
| Heavy chain variable region | QVQLKQSGAELVRPGASVKLSCKASGYTFTDYEMHWVKQTPVHGLEWIGALDP GTGDTAYKQKFKGKATLTADKSSSTAYMVLRSLTSEDSAVYYCTRGASYSNYE DYWGQGTSLTVSS | 1 |
| Light chain variable region | DVVMTQTPLTLSVTIGQPASISCKSSQSLLDSDGKTYLNWLLQRPGQSPKRLI YLVSKLDSGVPDRFTGSGSGTDITLRISTETQKMEFIIAGIIILIFRTRSEGGP SWKDGLM | 2 |

### 9. Evaluation of Antibody Titer by Flow Cytometry

An antibody titer was evaluated by flow cytometry using two types of HEK293T cells used in the panning operations. In the flow cytometry, CytoFLEX (Beckman Coulter) was used.

The cells were labeled as follows: 10-fold dilution series from 10 µg/mL to 1.0 ng/mL of each produced monoclonal antibody were added to the HEK293T cells of each type. Anti-Human IgG Goat pAbs PE (Cat. No: #IM0550; Beckman Coulter) were diluted 200-fold and used as a secondary antibody. A polyclonal rabbit anti-SYT2 N-terminal antibody (Polyclonal rabbit purified antibody SYT2 luminal domain; Cat. No. 105 223; Synaptic Systems) was used as a control primary antibody. Anti-Rabbit IgG(H+L)-PE(Cat. No: #IM0855; Beckman Coulter) was diluted 200-fold and used as a secondary antibody thereagainst.

For the antibody according to the present invention, strong fluorescence was detected at a low antibody concentration among the obtained monoclonal antibodies. In particular, fluorescence intensity at the antibody concentration of 0.01 µg/mL was about twice that of the control polyclonal antibody, suggesting that the monoclonal antibody according to the present invention is capable of efficiently binding even at low concentrations to the antigen and is a highly sensitive antibody.

### 10. Identification of Sequences of Complementarity Determining Regions (CDRs)

The amino acid sequences of the CDRs of the monoclonal antibody according to the present invention were identified according to the Kabat method.

The amino acid sequence of each CDR thus identified is shown in Table 4.

**[Table 4]**

| Table 4: Amino acid sequences of CDRs of the monoclonal antibody of the present invention | | | |
|---|---|---|---|
| | | Sequence | SEQ ID NO |
| Heavy chain | HCDR1 | TDYEMH | 3 |
| | HCDR2 | ALDPGTGDTAYKQKFKG | 4 |
| | HCDR3 | GASYSNYEDY | 5 |
| Light chain | LCDR1 | KSSQSLLDSDGKTYLN | 6 |
| | LCDR2 | LVSKLDS | 7 |
| | LCDR3 | FIIAGIILI | 8 |

### < Example 2: Evaluation of Monoclonal Antibody >

The monoclonal antibody obtained was evaluated for its binding characteristics against an antigen.

The antigen used was pan-hSYT2, and the binding characteristics of the antibody were measured using the Octet RED96e System (Sartorius). In the measurement, the following conditions were used:
biosensor : Streptavidin biosensor (Cat. No: #18-5019; Sartorius);
antibody concentration: 5 nM/2.5 nM/1.25 nM/0.63 nM/0.31 nM/0.17 nM/0.08 nM/0 nM;
buffer: PBS containing 0.002% Tween-20 and 0.01% BSA;
equilibration before immobilization: incubation for 60 seconds in buffer alone;
immobilization: incubation for 300 seconds in buffer containing the antigenic peptide;
equilibration before antibody addition: incubation for 180 seconds only in buffer alone;
antibody binding reaction: incubation for 600 seconds in buffer containing the antibody;
antibody dissociation reaction: incubation for 1800 seconds in buffer alone;
All the incubation operations were performed at a stirring rate of 1000 rpm at 30°C.

In the measurement using the polyclonal rabbit anti-SYT2 N-terminal antibody, the antigen was used at a concentration of 0.2 µg/mL. In contrast, when the monoclonal antibody according to the present invention was used in the measurement, the antigen was used at a concentration of 0.05 µg/mL.

The measurement results are shown in Tables 5 and 6. Table 5 shows the results obtained using the antigen at a concentration of 0.2 µg/mL, and Table 6 shows the results obtained using the antigen at a concentration of 0.05 µg/mL.

**Table 5: Measurement results about antigen used at concentration of 0.2 µg/mL**

| | Affinity (M) | Association rate constant (1/Ms) | Dissociation rate constant (1/s) |
|---|---|---|---|
| Control polyclonal antibody | 1.85 E-10 | 1.40 E+5 | 2.60 E-5 |

**[Table 6]**

| Table 6: Measurement results about antigen used at concentration of 0.05 µg/mL | | | |
|---|---|---|---|
| | Affinity (M) | Association rate constant (1/Ms) | Dissociation rate constant (1/s) |
| the monoclonal antibody of the present invention | 4.48 E-11 | 2.52 E+5 | 1.13 E-5 |

The affinity is a value obtained by dividing the dissociation rate constant, reflecting the ease of progression of the dissociation reaction, by the association rate constant, reflecting the ease of progression of the binding reaction, with a smaller value indicating higher affinity.

The results shown in Tables 5 and 6 revealed that the monoclonal antibody according to the present invention has a low dissociation constant compared to the results measured in the control polyclonal antibody, and indicating a lower likelihood of dissociation from the antigen and, overall, a high affinity.

### < Example 3: Verification of Pharmacological Effect by Delivery of Drugs Using Synaptotagmin 2 Antibody >

Using a polyclonal Synaptotagmin 2 antibody, it was examined whether a drug-based pharmacological effect was observed when a drug was delivered into the motor neuron.

### 1. Cell Culture

Human iPS-derived motor neuron (40HU-005-2M; ixcells biotechnologies) was thawed using Dead Cell Removal kit (Veritas) according to the protocol of the kit. The cells after thawing were plated in 96-well plates (V-bottom) at a density of 2×10⁴ cells/well and cultured in the motor neuron culture medium (Motor Neuron Maintenance Medium; ixcells biotechnologies) for 1 week to prepare neurospheres. The prepared neurospheres were selected based on size and roundness, and those satisfying this criterion were used in the subsequent experiments.

After coating a 96-well EZVIEW (registered trademark) culture plate LB (AGC Technoglass Co., Ltd.) with poly-D-lysine and Geltrex (registered trademark) Matrix (Thermo Fisher Scientific), the selected neurospheres were then plated and cultured for 20 days. As the medium, the above-described motor neuron culture medium was used first, and medium change with the same medium was performed on the second day of culture. Thereafter, medium was changed three times per week using neuronal medium (Neurobasal plus medium (B27 plus supplement (Thermo Fisher Scientific), 20ng/mL BDNF, 20ng/mL GDNF, and penicillin-streptomycin were added)). Each medium change was performed at 50 µL/well.

### 2. Preparation of LRRTM2 Bead

Microbeads coated with the extracellular domain of LRRTM2 (active beads) were prepared as disclosed in WO2021/006075.

Specifically, streptavidin-coated microbeads (Streptavidin Coated Microspheres; Bangs Laboratories, Inc.; polystyrene, mean diameter 9.94µm) were washed twice with wash buffer (phosphate buffered saline (PBS), 0.01% bovine serum albumin (BSA), 0.05% TritonX-100) and reacted with biotinylated anti-human IgG (Fc-specific) antibody (Sigma-Aldrich; mouse monoclonal) in binding buffer (PBS, 0.01% BSA) to immobilize the biotinylated anti-human IgG (Fc-specific) antibody on streptavidin-coated microbeads. The resulting beads were washed three times with wash buffer (anti-human IgG-Fc antibody beads).

Anti-human IgG-Fc antibody beads were then suspended in a binding buffer to which a fusion protein of the extracellular domain of human LRRTM2 and the Fc part of human IgG (LRRTM2-Fc; R&D systems) were added to immobilize LRRTM2-Fc onto the anti-human IgG-Fc antibody beads. The resulting beads were washed with wash buffer and suspended in binding buffer (LRRTM2 bead suspension).

### 3. Induction of Presynapse (Figure 1)

Plates cultured for 20 days were seeded with LRRTM2 beads at 0.1µg/well and cultured for 48 hours at 37°C to induce presynapse formation.

### 4. Production of Conjugate

As the drug, a microtubule polymerization inhibitor, monomethyl auristatin E (MMAE: MedChemExpress), was used. A conjugate of MMAE and the antibody was prepared using the MagicLink^{™} kit (broadpharm). Preparation of the conjugate was performed according to the manufacturer's protocols.

As an antibody, a polyclonal rabbit anti-SYT2 N-terminal antibody (Polyclonal rabbit purified antibody SYT2 luminal domain; Cat. No. 105 223; Synaptic Systems), or a normal rabbit IgG antibody (Normal Rabbit IgG; Cat. No. AB-105-C; R&D Systems), as a control, was used.

### 5. Preparation of Conjugate Solutions

After warming the neuronal medium at 37°C for 30 minutes, 4-aminopyridine (Sigma Aldrich) was added to the medium to a final concentration of 100µM and mixed. In addition, the conjugate was added to a final concentration of 1µg/mL and mixed. The crude conjugate solution was centrifuged at 200g for 3 minutes at room temperature, and the supernatant was collected as the conjugate solution.

As a further control group, MMAE alone, instead of conjugate, was introduced to some samples (sample size: 13). For this group, MMAE solution was prepared as described above, using MMAE instead of conjugate at the same concentration.

### 6. Introduction of the Conjugate

The conjugate was introduced into a plate, in which presynapse had formed, by changing media with 100µL of conjugate solution or MMAE solution and culturing at 37°C for 30 minutes.

### 7. Axonal Elongation Reaction

After the introduction, the solutions were collected and washed, and the medium was changed with neuronal medium lacking the conjugate and MMAE. The axons were then elongated by culturing for an additional 24 hours.

### 8. Fixation of Cells

After culturing, the cells were washed with neuronal medium and then fixed with 2% paraformaldehyde (PFA) solution.

### 9. Immunocytochemical Staining

Immunocytochemical staining was performed using the added antibody as the primary antibody. The cells after fixation were permeabilized with detergent and blocked, followed by a primary antibody reaction using a mouse anti-βIII-tubulin (Tuj1) antibody (Cat. No. 801202; Biolegend) as an additional primary antibody. A secondary antibody reaction was then performed using Alexa 555-labeled anti-mouse antibody (Cat. No. A32727; Thermo Fisher Scientific) to obtain fluorescent images. Blocking was performed using blocking buffer (PBS + 2% normal goat serum + 1% BSA + 1% fetal bovine serum + 0.02% TritonX-100). The sample sizes were as follows: the MMAE simple introduction group, 13 samples; the control antibody group, 17 samples; the polyclonal SYT2 antibody group, 27 samples.

Fluorescent images were obtained using an inverted live cell (DMi8) microscopic fluorescent microscope (Leica), equipped with LAS X software (Leica), at the following excitation wavelengths, detection wavelengths, exposure times, and detection thresholds. All gamma correction values used were 1.

Excitation wavelength and detection wavelength: Alexa 555 maximum excitation 555 nm; maximum detection 580 nm; actual detection 595 nm.
Exposure time and detection threshold: Alexa 555 exposure time 50 ms; detection threshold 100-3500.

### 10. Analysis of Axonal Amount

The axonal amount was obtained as the sum of the brightness (= (fluorescence intensity per pixel) × (pixel number)) of Tuj1 signals in the obtained fluorescence images. The relative amount of axon was calculated as the value normalized to the results obtained using the conjugate of the control normal rabbit antibody and MMAE set to 100%. Brightness was obtained using LAS X software (Leica).

### 11. Statistical Analysis

Statistical analysis was performed using a t-test using GraphPad Prism9 (GraphPad Software). The significance level used was p<0.05.

The results are shown in Figures 2 to 4. Figures 2 and 3 are immunocytochemical staining images showing the images of axons of the control antibody group (Figure 2), in which the conjugate of the control normal rabbit antibody and MMAE was used, and the SYT2 antibody group (Figure 3), in which the conjugate with polyclonal anti-SYT2 N-terminal antibody was used. In addition, Figure 4 is a graph showing the results quantitatively.

In all experimental conditions, axons were elongated from the neurospheres, and the presynapse was normally induced (Figures 2A and 3A).

MMAE inhibits the polymerization of microtubules, the cytoskeleton that is critical for axon elongation and maintenance. Therefore, it is expected that the stronger the effectiveness of MMAE, the more inhibited the elongation and maintenance of axons and resulting in a decrease in the axonal amount.

In the control antibody group, many thick axons and the presynapse, which are observed as bulges, were observed even in the distal portion distal to the neurosphere (Figure 2B). In addition, in the proximal portion relatively close to the neurosphere, the axons were observed to run in an aligned manner, in which the axons are almost parallel to each other (Figure 2C).

On the other hand, in the SYT2 antibody group, only a small number of thin axons were observed in the distal portion, distal to the neurosphere, and presynapse formation was poor (Figure 3B). In addition, in the proximal portion, relatively close to the neurosphere, the axon orientation was not aligned as in the control antibody group, and it was observed that the axon orientation was disordered (Figure 3C).

The quantitative results also supported these observations. Compared with the control antibody group ("Cont. IgG-MMAE" in Figure 4), the axonal amount was significantly decreased, and the relative amount of axon was about 87.15% in the polyclonal SYT2 antibody group ("α-SYT2 IgG-MMAE" in Figure 4). Additionally, the results of the SYT2 antibody group were significantly decreased, even compared with the standalone introduction group ("MMAE" in Figure 4), in which MMAE was introduced alone, and the axonal amount was about 82.7% of that of the standalone introduction group. There were no significant differences between the standalone introduction group and the control antibody group.

### < Example 4: Verification of Pharmacological Effect by Delivery of Drugs Using Monoclonal Antibody According to the Present Invention >

Using the monoclonal antibody according to the present invention obtained in Example 1, the effect on a drug-based pharmacological effect was examined where a drug was delivered into the motor neuron.

The same experiment as in Example 3 was conducted, except that the monoclonal antibody according to the present invention obtained in Example 1 was used in place of the anti-SYT2 N-terminal antibody, and the polyclonal rabbit anti-SYT2 N-terminal antibody was used instead of the control antibody. The experiment was conducted using 3 samples per group.

The results are shown in Figure 5. Figure 5 is a graph quantitatively showing the results obtained using the monoclonal antibody according to the present invention.

MMAE inhibits the polymerization of microtubules, the cytoskeleton that is critical for axon elongation and maintenance. Therefore, it is expected that the stronger the effectiveness of MMAE, the more inhibited the elongation and maintenance of axons and resulting in a decrease in the axonal amount.

The axonal amount was significantly decreased in the monoclonal SYT2 antibody group ("Monoclonal Ab" in Figure 5) compared with the polyclonal antibody group ("Polyclonal Ab" in Figure 5).

This reconfirmed that the delivery efficiency of the physiologically active substance depends on the properties of an antibody, and also suggested that the use of the monoclonal antibody according to the present invention, even compared to the polyclonal antibody already shown to have a pharmacological effect, enables the physiologically active substance or the like to be delivered significantly efficiently to the motor neuron.

### < Example 5: Confirmation of Affinity of Monoclonal Antibody According to the Present Invention by ELISA >

The first monoclonal antibody according to the present invention obtained in Example 1 was examined for its binding affinity to an antigen by ELISA using cultured cells expressing SYT2 on the cell membrane.

### 1. Preparation of Cell

SYT2-expressing cells (cells expressing the intravesicular domain of SYT2 on the cell surface) were prepared by commission to Medical & Biological Laboratories Co., Ltd. Preparation procedures are summarized as follows:

The cultured cells used were HEK293T cells (manufactured by American Type Culture Collection).

As an SYT2 expression vector, a plasmid vector (SEQ ID NO: 19) was used in which a DNA fragment with a gene sequence (SEQ ID NO: 21) encoding human SYT2 (having the amino acid sequence of SEQ ID NO: 9) was integrated into pCMV6-AC-Myc-DDK-IRES-GFP-Puro Mammalian Expression Vector plasmid (manufactured by OriGene).

The plasmid vector was introduced into the HEK293T cells as follows:

A solution for transfection was prepared by mixing 1 µL of a vector solution containing the plasmid vector at a concentration of 1 µg with 100 µL of Opti-MEM^{™} I Reduced Serum Medium (manufactured by Thermo Fisher Scientific).

As a medium for transfection, Opti-MEM^{™} I Reduced Serum Medium (manufactured by Thermo Fisher Scientific) supplemented with 2 µL of Lipofectamine^{™} 2000 Transfection Reagent (manufactured by Thermo Fisher Scientific) was used.

For transfection, the HEK293T cells were plated in 24-well plates (manufactured by Iwaki) at a cell density of 1.0 × 10⁵ cells/well and cultured until 80 to 90% confluence in a medium for maintenance (DMEM medium (manufactured by Thermo Fisher Scientific) supplemented with 1% Penicillin-Streptomycin (manufactured by Thermo Fisher Scientific), 10% Fetal Bovine Serum (manufactured by Cytiva), and 1% GlutaMAX^{™} I (manufactured by Thermo Fisher Scientific)). After the culture, the medium was removed and replaced with 1 mL of the medium for transfection. After the medium replacement, 100 µL of the solution for transfection was added thereto and incubated at 37°C for 3 days in the presence of 5% CO₂.

After the incubation, the medium was removed and replaced with 1 mL of an equilibrated medium for expression (DMEM medium (manufactured by Thermo Fisher Scientific) supplemented with 2.5 µg/µL Puromycin (manufactured by Thermo Fisher Scientific)). After the medium replacement, the HEK293T cells were cultured at 37°C for 3 days in the presence of 5% CO₂ and thereby allowed to express SYT2. The medium replacement was performed by the replacement of the whole amount every 3 days, and subculture was performed 3 days after the start of culture in the medium for expression.

### 2. Subculture

After the culture, the medium was removed, and the cells were washed with PBS. After the washing, 2 mL of 0.1% trypsin (manufactured by Nacalai Tesque, Inc.) was added thereto and incubated at room temperature for 1.5 minutes to 2 minutes to detach the cells, and the reaction was terminated by deactivating trypsin by pipetting. The cells were collected into a 50 mL tube, and cell masses were dispersed by pipetting.

A suspension of the dispersed cells was centrifuged at 130 × g at room temperature for 5 minutes to remove the supernatant, and the cells were suspended by the addition of 10 mL to 20 mL of the medium for expression. The number of cells was counted, and the cells were plated on 24-well plates at a cell density of 1.0 × 10⁵ cells/well.

### 3. ELISA Test

From the cells cultured for 3 days after the subculture, a cell suspension based on a medium for measurement (DMEM medium (manufactured by Thermo Fisher Scientific) supplemented with 1% Penicillin-Streptomycin (manufactured by Thermo Fisher Scientific), 10% Fetal Bovine Serum (manufactured by Cytiva), and 1% GlutaMAX^{™} I (manufactured by Thermo Fisher Scientific)) was obtained by the same procedures as in the subculture using the medium for measurement.

The obtained cell suspension was plated on 96-well plates for ELISA (manufactured by Iwaki) at a cell density of 2 × 10⁴ cells/well and incubated at 37°C for 1 day in the presence of 5% CO₂.

In 1.5 mL tubes, primary antibody solutions were prepared to a primary antibody concentration of 1 µg/mL, 500 ng/mL, or 100 ng/mL. The medium for measurement was used as a solvent, and the primary antibody used was the polyclonal rabbit anti-SYT2 N-terminal antibody, used as a control antibody, or the monoclonal antibody according to the present invention.

After the incubation, the medium was removed from the 96-well plates for ELISA, and 100 µL of each of the primary antibody solutions was added to each well and incubated at 37°C for 1 hour in the presence of 5% CO₂.

After the primary antibody reaction, the antibody solutions were removed, and each well was washed with 100 µL of the medium for measurement. Then, a 2% PFA solution was added to the cells, which were then fixed at room temperature for 15 minutes and permeabilized using PBS containing 0.25% TritonX-100.

The cells thus permeabilized were washed with PBS, and blocking buffer (PBS + 2% normal goat serum + 1% BSA + 1% fetal bovine serum + 0.02% TritonX-100) was added to each well, and blocking was performed at room temperature for 1 hour.

After the blocking, a secondary antibody solution was added thereto and incubated at room temperature for 1 hour. The secondary antibody solution was prepared using the blocking buffer as a solvent. The secondary antibody used against the polyclonal rabbit anti-SYT2 N-terminal antibody was Goat anti-Rabbit 488 (Cat. No. #A32731; Thermo Fisher Scientific), and the secondary antibody used against the monoclonal antibody according to the present invention was Goat anti-Human 488 (Cat. No. #A48276; Thermo Fisher Scientific). The secondary antibody solution was supplemented with DAPI (Cat. No. D1306; Thermo Fisher Scientific) for each staining.

After the secondary antibody reaction, the antibody solutions were removed, and each well was washed with PBS containing 0.02% TritonX-100. Then, a 1% PFA solution was added to the cells, which were then postfixed at room temperature for 5 minutes. The fixative was then replaced with PBS.

### 4. Detection and Measurement of Fluorescence Signal

Fluorescent signals were measured using Promega GloMax (manufactured by Promega Corporation) with the following excitation wavelengths and detection wavelengths:
DAPI: excitation wavelength 365 nm, fluorescence wavelength 415 nm-445 nm, detection wavelength 475 nm
Alexa 488: excitation wavelength 475 nm, fluorescence wavelength 500 nm-550 nm, detection wavelength 525 nm

The fluorescence intensity of the detected Alexa 488 signals was divided by the fluorescence intensity of DAPI, and the obtained value was used as the relative fluorescence intensity for the anti-SYT2 antibody. The value normalized to the relative fluorescence intensity obtained using the control antibody, which was set to 1.0, was taken as the relative amount of antibody.

The results are shown in Figures 6 and 7. Figure 6 is a graph showing the results obtained using 100 ng/mL anti-SYT2 antibody, and Figure 7 is a graph showing the results obtained using 500 ng/mL anti-SYT2 antibody.

The antibody according to the present invention, even when used at a concentration as low as 100 ng/mL, had significantly strong binding ability to SYT2 compared to the control antibody.

From this result, the antibody according to the present invention was confirmed to be an excellent antibody, consistent with the results of the evaluation of the affinity of the antibody conducted in Example 2.

All publications, patents and patent applications cited in the description are incorporated in their entirety by reference.

## Claims

1. An antibody capable of binding to the intravesicular domain of Synaptotagmin 2, the antibody comprising
a heavy chain variable region comprising HCDR1 consisting of the amino acid sequence of SEQ ID NO: 3, HCDR2 consisting of the amino acid sequence of SEQ ID NO: 4, and HCDR3 consisting of the amino acid sequence of SEQ ID NO: 5, and
a light chain variable region comprising LCDR1 consisting of the amino acid sequence of SEQ ID NO: 6, LCDR2 consisting of the amino acid sequence of SEQ ID NO: 7, and LCDR3 consisting of the amino acid sequence of SEQ ID NO: 8.

2. The antibody according to claim 1, wherein the antibody is selected from the following (a) to (c):
(a) an antibody having the heavy chain variable region consisting of the amino acid sequence having the sequence identity of 90% or more to SEQ ID NO: 1, and the light chain variable region consisting of the amino acid sequence having the sequence identity of 90% or more to SEQ ID NO: 2;
(b) an antibody having the heavy chain variable region consisting of the amino acid sequence having substitution, deletion, and/or addition of one or several amino acids in SEQ ID NO: 1, and the light chain variable region consisting of the amino acid sequence having substitution, deletion, and/or addition of one or several amino acids in SEQ ID NO: 2; and
(c) an antibody having the heavy chain variable region consisting of the amino acid sequence of SEQ ID NO: 1, and the light chain variable region consisting of the amino acid sequence of SEQ ID NO: 2.

3. A conjugate of the antibody according to claim 1 and a label substance and/or a physiologically active substance.

4. A nucleic acid molecule having a nucleotide sequence encoding the antibody according to claim 1.

5. A cell comprising the nucleic acid molecule according to claim 4.

6. A targeting agent to a motor neuron comprising the antibody according to claim 1.

7. The targeting agent according to claim 6, further comprising a label substance and/or a physiologically active substance.

8. The targeting agent according to claim 7, comprising a conjugate of the antibody and the label substance and/or the physiologically active substance.

9. The targeting agent according to claim 7, wherein the label substance is a fluorescent molecule.

10. The targeting agent according to claim 7 comprising the label substance, wherein the targeting agent is a motor neuron visualizing agent.

11. The targeting agent according to claim 7, wherein the physiologically active substance is one or more selected from the group consisting of a synaptogenesis-promoting agent, a synapse-maintaining agent, a muscle-enhancing agent, and a neuronal function-modifying agent.

12. The targeting agent according to claim 6, wherein the targeting agent is incorporated intracellularly by endocytosis.

13. A pharmaceutical composition comprising the targeting agent according to claim 7.

14. A method for targeting the label substance and/or the physiologically active substance to a motor neuron comprising a step of contacting the targeting agent according to claim 7 and/or the pharmaceutical composition according to claim 13 with a motor neuron; and a step of delivering the targeting agent and/or the pharmaceutical composition to a synapse of the motor neuron.

15. A method for visualizing a motor neuron comprising a step of contacting the targeting agent, which is the motor neuron visualizing agent according to claim 10, with a motor neuron; a step of delivering the targeting agent to a synapse of the motor neuron; and a step of detecting a signal of the label substance.
